# EUROPEAN PATENT APPLICATION

(11) **EP 4 227 401 A1**
(43) Date of publication of application: **16.08.2023**
(21) Application number: 22156361.2
(22) Date of filing: 11.02.2022
(51) Int. Cl.: C12N 5/00, C12N 5/09

(54) **AN EX VIVO TUMOUR IMMUNE MICROENVIRONMENT MODEL, A METHOD FOR PRESERVING A TUMOUR-SPECIFIC IMMUNE CELL PROFILE IN AN EX VIVO TUMOUR IMMUNE MICROENVIRONMENT MODEL AND USE OF NANOFIBRILLAR CELLULOSE**

(71) Applicant: UPM-Kymmene Corporation, 00100 Helsinki (FI)
(72) Inventor: Nuopponen, Markus, 00100 Helsinki (FI); Mikkonen, Piia, 00100 Helsinki (FI); Sheard, Jonathan, Chaddesley Corbett, DY10 4SG (GB); Klefström, Juha, 00100 Helsinki (FI); Munne, Pauliina, 00100 Helsinki (FI); Turpin, Rita, 00100 Helsinki (FI); Peura, Aino, 00100 Helsinki (FI); Tervonen, Topi, 00100 Helsinki (FI); Salmela, Maria, 00100 Helsinki (FI)
(74) Representative: Berggren Oy

(57) **Abstract**

The present disclosure provides ex *vivo* tumour immune microenvironment model comprising patient-derived explant with a tumour-specific immune cell profile embedded in a matrix comprising nanofibrillar cellulose hydrogel having a concentration in the range of 0.25-1.2% by weight, wherein the nanofibrillar cellulose comprises fibrils and/or fibril bundles having number-average diameter of 200 nm or less. The present disclosure also provides a method for preserving a tumour-specific immune cell profile in an *ex vivo* tumour immune microenvironment model, the method comprising providing a patient-derived explant with a tumour-specific immune cell profile, providing a matrix comprising nanofibrillar cellulose hydrogel, wherein the nanofibrillar cellulose comprises fibrils and/or fibril bundles having number-average diameter of 200 nm or less, embedding the patient-derived explant with a tumour-specific immune cell profile in the matrix comprising nanofibrillar cellulose hydrogel, to obtain the ex *vivo* tumour immune microenvironment model, wherein the nanofibrillar cellulose hydrogel has a concentration in the range of 0.25-1.2% by weight. The model and the method are useful for example in drug discovery. The present disclosure also provides use of nanofibrillar cellulose.

## Description

### Field of the application

The present application relates to ex *vivo* tumour immune microenvironment models and methods for providing such models.

### Background

Biomimetic ex *vivo* culture systems for patient-derived tumour tissues hold great promise for cancer research by providing an attractive alternative for reductionist and artifact-prone cancer cell lines and models. The ex *vivo* culture system provides a source of patient-specific molecular information, which can be used for testing different treatment options with respect to obtained molecular information.

The present 3D cancer models lack important cell types such as immune cells or fibroblasts, which together with the tumour cells form an entity, known as tumour microenvironment. The complex interactions between the tumour and its microenvironment plays a fundamental role in the regulation of treatment responses. Therefore, it is important to maintain the biological context in such models and provide such conditions that facilitate the viability and maintenance of not only the tumour cells but also its microenvironment, as well as the interactions between these two. This has been found very challenging, especially for human cells.

Due to the reductionist 3D models, which lack the immune cell contexture, the ex *vivo* immunotherapy efficacy studies have been impossible. There is a growing need to identify the unifying features and critical differences that define distinct classes and subclasses of tumour immune microenvironment (TIME), which relate to the likelihood of response to immunotherapeutics. There also exists a need for methods, models and scaffolds, which can preserve the tumour immune microenvironment and enable determining how cancer patients will respond to treatment and if the immune system will help or hinder the treatment response. This would have tremendous value for cancer drug discovery and developers of immunotherapies.

### Summary

It was found out how to provide an *ex vivo* tissue model system preserving the tumour immune microenvironment (TIME) and which could overcome prior art issues.

The present disclosure provides an *ex vivo* tumour immune microenvironment model comprising patient-derived explant with a tumour-specific immune cell profile embedded in a matrix comprising nanofibrillar cellulose hydrogel having a concentration of the nanofibrillar cellulose in the range of 0.25-1.2% by weight, wherein the nanofibrillar cellulose comprises fibrils and/or fibril bundles having number-average diameter of 200 nm or less, such as 100 nm or less.

The present disclosure also provides a method for preserving a tumour-specific immune cell profile in an ex *vivo* tumour immune microenvironment model, the method comprising
- providing a patient-derived explant with a tumour-specific immune cell profile,
- providing a matrix comprising nanofibrillar cellulose hydrogel, wherein the nanofibrillar cellulose comprises fibrils and/or fibril bundles having number-average diameter of 200 nm or less, such as 100 nm or less,
- embedding the patient-derived explant with the tumour-specific immune cell profile in the matrix comprising nanofibrillar cellulose hydrogel,
to obtain the ex *vivo* tumour immune microenvironment model, wherein the nanofibrillar cellulose hydrogel has a concentration of the nanofibrillar cellulose in the range of 0.25-1.2% by weight.

The present disclosure also provides use of nanofibrillar cellulose comprising fibrils and/or fibril bundles having number-average diameter of 200 nm or less, such as 100 nm or less, for preparing an ex *vivo* tumour immune microenvironment model comprising patient-derived explant with a tumour-specific immune cell profile embedded in a matrix comprising the nanofibrillar cellulose hydrogel.

The main embodiments are characterized in the independent claims. Various embodiments are disclosed in the dependent claims. The embodiments and examples disclosed herein are mutually freely combinable unless otherwise explicitly stated.

The present model was able to maintain original properties of the tumours, such as histological features, phenotype, gene expression profile, and immune cell composition, which are prone to change in *ex vivo* conditions.

The present disclosure demonstrates the suitability of nanocellulose-based matrices for immuno-oncology purposes first with peripheral blood mononuclear cells (PBMC), and then with the patient-derived explant cultures (PDECs) platform. Each of the studied matrices could support immune cell survival without the additional supplementation of IL-2, which is normally needed in their conventional growth at the suspension. However, the tested matrices also enable functionalization with biotinylated recombinant IL-2 to support immune cell survival and particularly the maintenance of tumour infiltrating lymphocytes. The ability to functionalize the matrix may also enable differentiating other cell types in the tumour microenvironment.

The PDECs are unique ex *vivo* cancer models compared to many rivalling 3D culture systems since the PDECs preserve the original immuno-contexture of the tumour they were originally derived from. This means that the quantity and quality of the immune cells present in the PDECs reflect the original status of the immune cells well. Since the tumour-infiltrating lymphocytes (TILs) are the main targets of immune checkpoint inhibitors such as anti-PD-1 and anti-PD-L1, the PDECs that preserve the tumour immune microenvironment (TIME) have tremendous value for developers of immune oncology (IO) diagnostics and immuno-therapies. That means, for practically all major pharmaceutical companies in the world. Despite the efficacy of the immune therapies, not all tumours are suited for the treatment due to the lack of immune cells. Cancer immune evasion means that the cancer cells can hide from the immune cell surveillance for an unknow reason. To overcome the immune evasion remains as major challenge in designing effective anticancer therapeutic modalities. The present study demonstrates the suitability of cellulose matrices for immune oncological studies.

Recombinant proteins are expensive and are unstable after reconstitution, which makes the immune cell *in vitro* cultures highly costly. Using NFCs to substitute the use of IL-2 supplementation can provide a novel market opportunity for NFCs in immunological research. Linking IL-2 into a small volume of matrix is much more cost efficient than adding the protein into a large volume of media, as is done with conventional culture methods.

All tested nanocellulose matrices were able to maintain the immune cell viability and activation at least for one week in the culture. The sample viability was comparable to uncultured control samples and the prior art animal-based ECM matrix materials.

Differences in the immune cell activity between different matrices were seen, which may be utilized in gaining insight into the tumour-associated and infiltrating immune cells. The matrix stiffness was shown to have importance in the activity of tumour resident immune cells.

It has been recently acknowledged that a patient's own immune system can recognize and eliminate tumour cells. Therefore immune-based cancer treatments can be developed, but the challenge has been to obtain physiologically relevant 3D culture models to study the mechanisms involved, such as to model the ability of immune cells to infiltrate, recognize and eliminate tumour cells.

Especially human cells that are sensitive and require certain pressure to survive and proliferate, could be supported by the used matrix. Without binding to a theory it is believed that the high aspect ratio of the highly fibrillated cellulosic nanofibrils and/or fibril bundles providing a high number of exposed hydroxyl groups and the specific chemical environment in the matrix could create such conditions, which facilitated the maintenance of different biological substances at the same time, including tumour tissue and sensitive immune cells.

The findings suggest that the present nanofibrillar cellulose matrices can provide the desired properties at a certain concentration range, which is usually lower compared to prior art materials, and the nanofibrillar cellulose matrix can also exhibit good permeability at said concentration, which is required for efficient use of the obtained *ex vivo* model system in modelling applications, for example when substances such as drug candidate molecules are provided to the system. Especially the present matrices provide suitable conditions to maintain and activate the enclosed immune cells, and to allow flow of the substances immediately to the immune cells, which further enables obtaining fast and reliable response, and detecting the response.

Features of nanofibrillar cellulose contributing these conditions were found to include concentration, chemical modification type and degree, fibril dimensions, aspect ratio, source of cellulose, functionalization and further processing of the cellulose during manufacture of the nanofibrillar cellulose. Not all fibrillar celluloses could provide similar conditions.

Highly fibrillar nanofibrillar celluloses, especially anionic types, were found to be advantageous in many respects. In addition to the mechanical, chemical and functional properties the nanofibrillar celluloses with low fibril diameter provide transparency, which is advantageous when carrying out examination procedures requiring visual or optical examination on the model.

When nanofibrillar cellulose was used as matrix material, it could be functionalized with lower amount of functional molecules compared to prior art materials or compared to an option wherein the functional molecule is added in liquid medium, to obtain a similar effect. The difference of required functional molecules could be 10-100 fold or more, so with the present systems and materials and present conjugation and functionalization it was possible to use significantly lower amount of expensive bioactive (functional) substances for obtaining efficient functionality. In the present ex *vivo* model it is also not necessary to supplement the functional substances into the medium in high volumes and/or amounts and/or continuously, which helps keeping the model system relatively compact and simple. It may not be necessary to provide functional substances, such as cytokines, growth factors and the like at all in the culture medium. Especially it was found out that commonly used interleukine 2 supplementation was not necessary.

The present nanofibrillar cellulose matrix materials are preferably derived from plant origin and are thus substantially more of uniform quality and less expensive compared to prior art materials based on animal-derived materials. With the present materials very large systems with high matrix volumes can be implemented cost-efficiently. No biologically active compounds are present which could harmfully affect the proliferation and/or maintenance of the explant culture or using the model. Compared to conventional floating cultures the present model provides a stable, inexpensive and controllable option.

The present ex *vivo* model can be used to mimic the immuno-oncological properties of native tissues. The model can facilitate working with 3D cultures in high throughput. The model may be used in a wide range of applications, for example in disease modelling, drug screening and development and designing of complex tissue constructs. It may provide greater understanding of the molecular mechanisms underlying immune suppression in the tumour immune microenvironment, and evolving from more general one-size-fits-all platforms to specialized models tailored to individual questions.

### Brief description of the figures

Figure 1 shows immunofluorescent staining of PDECs at day 7 in 0.3% NFC. Green staining shows proliferation (KI67) or cell death (CC3). Red staining indicates cell cytoskeleton and blue staining shows cell nuclei. Scale bar 10 µm. FoV: field of view.
Figure 2 shows immunofluorescent staining of PDECs at day 7 in 1.0% NFC. Green staining shows proliferation (KI67) or cell death (CC3). Red staining indicates cell cytoskeleton and blue staining shows cell nuclei. Scale bar 10 µm. FoV: field of view.
Figure 3 shows immunofluorescent staining of PDECs at day 7 in 0.3% ANFC. Green staining shows proliferation (Ki67) or cell death (CC3). Red staining indicates cell cytoskeleton and blue staining shows cell nuclei. Scale bar 10 µm. FoV: field of view.
Figure 4 shows immunofluorescent staining of PDECs at day 7 in 0.7% ANFC. Green staining shows proliferation (Ki67) or cell death (CC3). Red staining indicates cell cytoskeleton and blue shows cell nuclei. Scale bar 10 µm. FoV: field of view.
Figure 5 shows immunofluorescent staining of PDECs at day 7 in 0.3% ANFC-A/IL2. Green staining shows proliferation (KI67) or cell death (CC3). Red staining indicates cell cytoskeleton and blue staining shows cell nuclei. Scale bar 10 µm. FoV: field of view.
Figure 6 shows immunofluorescent staining of PDECs at day 7 in 0.7% ANFC-A/IL2. Green staining shows proliferation (KI67) or cell death (CC3). Red staining indicates cell cytoskeleton and blue staining shows cell nuclei. Scale bar 10 µm. FoV: field of view.
Figure 7 shows immunofluorescent staining of PDECs at day 7 in Matrigel [10 mg/ml]. Green staining shows proliferation (KI67) or cell death (CC3). Red staining indicates cell cytoskeleton and blue staining shows cell nuclei. Scale bar 10 µm. FoV: field of view.
Figure 8 shows immunofluorescent staining of PDECs at day 0, before the 3D culturing. Green staining shows proliferation (KI67) in different concentrations of NFC matrices. Red staining indicates cell cytoskeleton and blue staining shows cell nuclei. Scale bar 10 µm. FoV: field of view.
Figure 9 shows immunofluorescent staining of PDECs at day 7. Green staining shows macrophages (CD11b) and yellow staining indicates lymphocytes. Red staining indicates cell cytoskeleton and blue staining shows cell nuclei. Scale bar 10 µm.
Figure 10a shows flow cytometry quantification of the total number of cells - results from PBMCs grown in Matrigel and NFCs for 5 days. The size of the ball and colour indicates the cell numbers in microliter. The figure shows T-cells, Monocytes, Lymphocytes, B-cells/progenitor cells, and all live cells.
Figure 10b shows flow cytometry results from PBMCs grown in Matrigel and NFCs for 5 days. The size of the ball and colour indicates the cell numbers in microliter. The figure shows NK- and NKT- cells with and without activity marker CD16.
Figure 10c shows flow cytometry results from PBMCs grown in Matrigel and NFCs for 5 days. The size of the ball and colour indicates the cell numbers in microliter. Marker show: More CD80 is expressed on macrophages and Denditic cells (DC). More CD86 is expressed on monocytes and DCs. CD14 is a monocyte and macrophage marker.
Figure 11 shows flow cytometry results from PBMCs grown in NFCs after 5 days of culture. The data is the same that in the Figure 10, but each of the cell types are compared to the live cell number. Each graph shows the same markers that are in the Figure 10. The number inside the ball show the % of each immune cell type out of the total live immune cells in each matrix. Different colours represent different immune cell markers.
Figure 12 shows flow cytometry results from TIME-PDECS grown in Matrigel and 0.3% NFCs for 7 days. Results are shown as cells/µl. More intense red colour and bigger the ball size indicates more immune cells in that category.

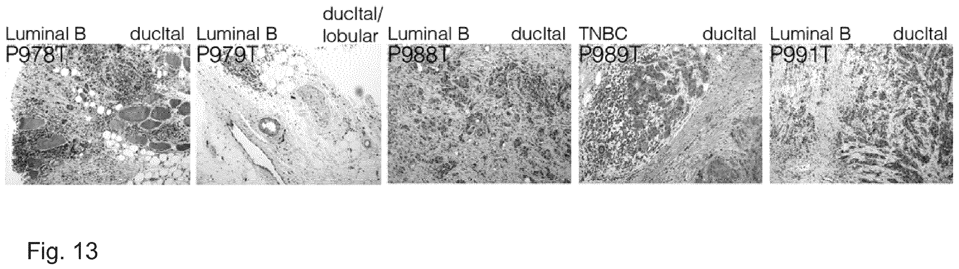

Figure 13 shows immunohistology images of the tumour samples, which were used in the FACS analysis in Figure 12. The samples show differences in the immune cell contexture between the samples. The samples are stained with H&E.
Figure 14 shows cytokine assay results from PDECs grown in Matrigel and NFCs for 3 days. Media was collected and analysed with ELISA-based assay. The measured cytokine levels (pg/ml).

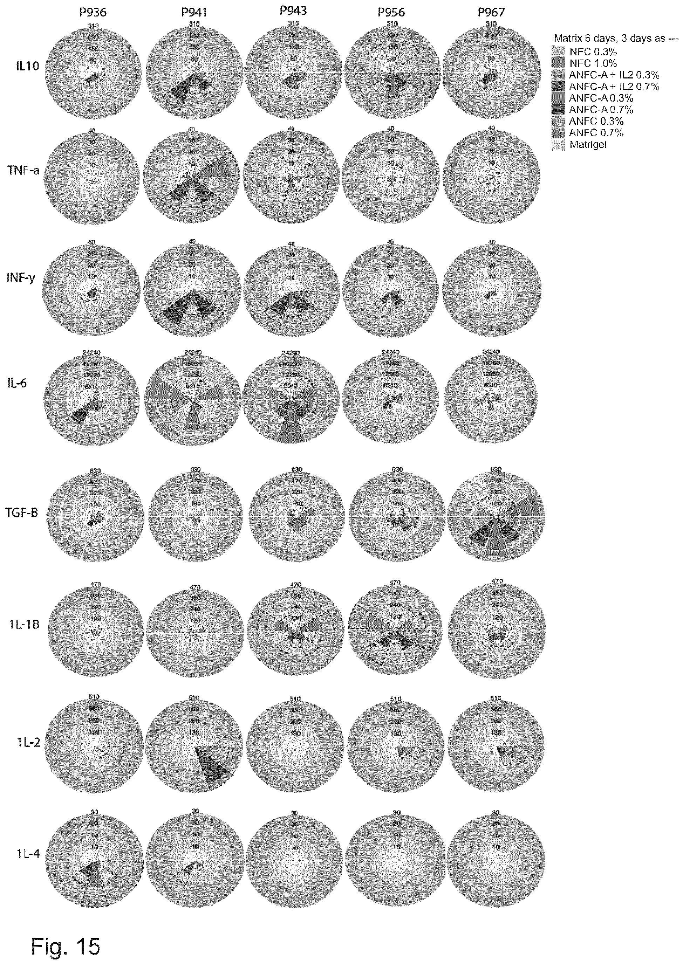

Figure 15 shows cytokine assay results from PDECs grown in Matrigel and NFCs for 3 and 6 days. The results from day 3 are marked with a dotted line and the results from the day 6 are shown with the full colour. The measured cytokine levels (pg/ml).
Figure 16 shows IL-2 cytokine levels (pg/ml) measured with ELISA-based assay from A. media of NFC-PDECs, B. media of NFC cell-free sample and C. media of NFC-PBMCs.
Figure 17 shows cytokine assay results from PBMCs grown in Matrigel and NFCs for 5 days. Media was collected at day 2 and day 5, and analysed with ELISA-based assay. A. IFN-γB. IL-10 and C. IL-4 cytokine levels (pg/ml).
Figure 18 shows cytokine assay results from PBMCs grown in Matrigel and NFCs for 5 days. Media was collected at day 3 and day 5, and analysed with ELISA-based assay. A. TNF-αB. IL-1βC. IL-6 and D. LAP (TGF-β1). cytokine levels (pg/ml).
Figure 19 shows the effect from the biotin supplementation on the inflammatory response in PBMCs (A) and PDEC (B). Cytokine assay results from PDECs grown in Matrigel and 0.3% NFCs for 7 days. Media was collected at day 7 and analysed with ELISA-based assay. A. IFN-γ and B. IL-10 cytokine levels (pg/ml).
Figure 20 shows PCA from 5 PDEC samples. A) Samples clustered according to their RNA quality. B) Sample clustering according to the patient. C) Sample clustering according to the culture condition. Group 1: uncultured, Group 2: Matrigel, Group 3: NFC 0.3%, Group 4: NFC 1.0%, Group 5: ANFC 0.3%, Group 6: ANFC 0.7%, Group 7: ANFC-A 0.3%, Group 8: ANFC-A 0.7%, Group 9: ANFC-A 0.3% + IL2, Group 10: ANFC-A 0.7% + IL2.
Figure 21 shows the scRNASEQ data from the uncultured patient samples (two patients pooled together). The immune cell subtypes are show in the plot with different colours (left). The expression of typical marker genes for different immune cell types are shown in pink (right).
Figure 22 shows the scRNASEQ data from the uncultured patient samples.The different colours indicate the different immune cell subtypes.
Figure 23 shows the scRNASEQ data from the uncultured and NFC cultured patient samples. The different colours indicate the different immune cell subtypes. The data is unpooled.
Figure 24 shows comparison of the scRNASeq data between 3d cultured and uncultured sample. Two patient samples are pooled together.
Figure 25 shows proportions of the immune cell types in the uncultured and 3D cultured samples. Two patient samples are pooled together. 25A: NFC, 25B: Uncultured.
Figure 26 shows gating strategy to identify lymphocyte subgroups. Lymphocyte population is recognized based on size on SSC-A vs FSC-A dot blot. T-cells were identified as CD3 positive cells, B-cells as CD19 positive cells and NK-cells as CD3 negative and CD56 positive cells. T-cell subgroups were identified as CD4 positive (T-helper), CD8 positive (T-cytotoxic) and CD56 positive (NKT-cells). Example of NFC 0.3% matrix is shown.
Figure 27 shows gating strategy to identify activated T-cells and NKT-cells (CD69 high). Examples from NFC 0.3% matrix and IL-2 conjugated ANFC-A 0.3% matrix are shown.
Figure 28 shows gating strategy to identify myeloid cells. Myeloid cells are gated based on size on SSC-A vs FSC-A dot blot, and express high CD80 and CD86. From myeloid cell population, monocytes are recognized as CD14 high, and macrophages and dendritic cells as CD14 low. M1 class macrophages express high CD16. Example of NFC 0.3% matrix is shown.
Figure 29 shows gating strategy to identify activated NK-cells (CD16 low). Examples from NFC 0.3% matrix and IL-2 conjugated ANFC-A 0.3% matrix are shown.
Figure 30 shows gating strategy to identify lymphocyte and monocyte populations in TIME-PDECs. Examples from NFC 0.3% matrix are shown.

### Detailed description

In this specification, percentage values, unless specifically indicated otherwise, are based on weight (w/w, by weight, or wt%). If any numerical ranges are provided, the ranges include also the upper and lower values. The open term "comprise" also includes a closed term "consisting of" as one option. The diameters disclosed herein, unless specifically indicated otherwise, refer to the smallest diameter, and may be presented as average or number-average diameter and may be determined microscopically.

The materials and products described herein may be medical and/or scientific materials and products, such as life science materials and products, and may be used in the methods and the applications involving living cells and/or bioactive material or substances, such as described herein, such as examination methods, cancer research, drug research and/or discovery, biomarker studies, signalling studies, diagnostic methods and the like methods and/or studies.

The present disclosure presents an ex *vivo* tumour immune microenvironment model comprising patient-derived explant with a tumour-specific immune cell profile. The present disclosure also presents a method for preserving a tumour-specific immune cell profile in an *ex vivo* tumour immune microenvironment model. The ex *vivo* tumour immune microenvironment model may be obtained with the method.

In general microenvironment refers to cells, molecules and structures that surround and support other cells and tissue. "Tumour microenvironment" (TME) refers to a complex and dynamic ensemble of proliferating tumor cells/stroma, blood vessels, infiltrating immune cells, and a variety of other tissue-associated cells. In the tumour microenvironment, cells not only interact with other cells of the same or different origin but are also rooted in the extracellular matrix.

The term "tumour immune microenvironment" (TIME) refers to immune components that exist within a patient's tumour, i.e. which are associated or resident with the tumour. An immune microenvironment comprises innate immune cells, adaptive immune cells, extracellular immune factors, and cell surface molecules. The TIME and its components may vary widely during neoplastic progression and among different patients. According to one definition the tumour immune microenvironment refers to the different subpopulations of the immune system and their interactions within a tumour microenvironmental niche, that have a unique influence on the tumour initiation, development and response to therapies.

In general, in science, *ex vivo* refers to experimentation or measurements carried out in or on tissue from an organism in an external environment with minimal alteration of natural conditions. *Ex vivo* conditions allow experimentation on an organism's cells or tissues under more controlled conditions than is possible in *in vivo* experiments. *Ex vivo* may not be equivalent to *in vitro.* According to one definition the term ex *vivo* refers to a situation wherein the samples to be tested have been extracted from an organism, such as in the case of patient-derived explants. *In vitro* method usually use a specific lineage of cells, that are isolated, separated and purified from their usual biological surroundings, *i.e.* created artificially.

Explant culture is a technique to organotypically culture cells from a piece or pieces of tissue or organ removed from an animal. The term explant can be applied to samples obtained from any part of the organism. In the present context the term is used to refer to tissue comprising primary and/or tumour cells obtained from a source, such as a patient, and/or to cells derived from said tissue, and the present model may represent an explant culture.

The term "patient-derived explant" (PDE) refers to a piece or fragment of tissue, such as tumour tissue, which is freshly obtained, such as surgically resected, from a patient. The explant may be immediately used in the present methods without freezing but it was also found out that the explants tolerated freezing and could be revived when embedded in the present matrices. PDEs generally involve the ex *vivo* culture of fragments of resected human tumours that retain the histological features of original tumours.

The patient may be a human patient. The patient may have a medical condition, such as a disease or a disorder, for example cancer or other tumour, or the patient may be suspected to have such a medical condition.

The present disclosure provides a method for preserving a tumour-specific immune cell profile in an *ex vivo* tumour immune microenvironment model, the method comprising providing a patient-derived explant with a tumour-specific immune cell profile.

Preserving refers to maintaining the composition, activity, viability, context and/or other properties of the immune cell profile in the ex *vivo* tumour immune microenvironment model. It is desired that the tumour-specific immune cell profile is preserved for a time period required for utilizing the model, such as at least 24 hours, at least 2 days, at least three days or at least 7 days. Preserving may also refer to maintaining the corresponding features of the tumour tissue or tumour cells.

Maintaining may comprise maintaining cell and/or tissue phenotype and/or cell and/or tissue identity, and/or maintaining the presence and/or activity of one or more features and/or elements of the cells and/or tissue, such as one or more receptors, one or more gene expression or products thereof, and the like. Maintaining may comprise maintaining or preserving 100% or substantially 100% of the level of the original activity or other property to be maintained, or maintaining or preserving at least 95%, 90%, 80%, 70%, 60% or 50% of the original. The percentage may be defined by comparing to a provided sample obtained directly from the tissue and carrying out a suitable method for detecting the activity or other property, for example a number, a level or an amount of a substance or an element of a cell.

The present model and methods using the model are herein explained in context of tumour tissue. However, the model and the methods can be applied to other tissues and immune cells in analogous way, where applicable. A model comprising patient-derived immune cells, or immune profile, can be obtained and maintained even without tumour or other tissue.

Disclosed is also an *ex vivo* immune microenvironment model comprising patient-derived immune cell profile, such as comprising peripheral blood mononuclear cells (PBMC), embedded in a matrix comprising a nanofibrillar cellulose hydrogel having a concentration in the range of 0.25-1.2% by weight, and a method for preserving an immune cell profile in an *ex vivo* immune microenvironment model, the method comprising providing a patient-derived immune cell profile, providing a matrix comprising nanofibrillar cellulose hydrogel, and embedding the patient-derived immune cell profile in the matrix comprising nanofibrillar cellulose hydrogel. Also disclosed is use of nanofibrillar cellulose for preparing such a model. The present description of the *ex vivo* tumour immune microenvironment model can be applied to the immune microenvironment model.

One example discloses use of nanofibrillar cellulose comprising fibrils and/or fibril bundles having number-average diameter of 200 nm or less, such as 100 nm or less, for preparing an *ex vivo* immune microenvironment model comprising a patient-derived immune cell profile of peripheral blood mononuclear cells embedded in a matrix comprising a nanofibrillar cellulose hydrogel having a concentration of the nanofibrillar cellulose in the range of 0.25-1.2% by weight.

The term "tissue" herein may be considered encompassing solid tissue and optionally any separate corresponding tissue cells, which may have been separated during taking and/or processing the sample. The tissue sample comprises or is an ensemble of two or more, such as a plurality, of cells which may be attached together substantially as they were in the original tissue with their stromal component and/or extracellular matrix from the same origin.

The sample is obtained from a tissue, which may be primary tissue and/or the tissue cells may comprise primary cells, and/or the tissue may be tumour tissue and/or the tissue cells may comprise tumour cells. The tumour may be cancer, so the tissue may be cancer tissue or cancerous tissue. The tumour may be also a benign tumour. Cancer as used herein may refer to any abnormal cell growth, especially with a potential to invade or spread to other parts in a body. Examples of cancer types include carcinomas, sarcomas, leukemias, lymphomas, myelomas, melanomas, brain and spinal cord tumours, germ cell tumours, neuroendocrine tumours, carcinoid tumours, and blastomas. Examples of specific cancers include for example breast cancer, prostate cancer and ovarian cancer.

The tissue may also be non-cancerous or non-tumorous tissue. In such case the tissue may exhibit properties that are otherwise interesting, and especially which are difficult to maintain in *ex vivo* conditions, such as cell phenotype or other characteristics.

The method comprises
- providing a patient-derived explant with a tumour-specific immune cell profile,
- providing a matrix comprising nanofibrillar cellulose hydrogel, wherein the nanofibrillar cellulose comprises fibrils and/or fibril bundles having number-average diameter of 200 nm or less, such as 100 nm or less,
- embedding the patient-derived explant with the tumour-specific immune cell profile in the matrix comprising nanofibrillar cellulose hydrogel,
to obtain the *ex vivo* tumour immune microenvironment model, wherein the nanofibrillar cellulose hydrogel has a concentration of the nanofibrillar cellulose in the range of 0.25-1.2% by weight.

An *ex vivo* tumour immune microenvironment model is obtained comprising patient-derived explant with a tumour-specific immune cell profile embedded in a matrix comprising nanofibrillar cellulose hydrogel having a concentration of the nanofibrillar cellulose in the range of 0.25-1.2% by weight, wherein the nanofibrillar cellulose comprises fibrils and/or fibril bundles having number-average diameter of 200 nm or less, such as 100 nm or less.

PDEs can be generated by fragmentation or slicing of fresh tumour tissue sample. The tissue sample may be treated and/or processed, such as chemically and/or enzymatically treated, such as digested with one or more enzymes, for example collagenases. The chemical and/ or enzymatic treatment may be carried out to digest at least partly bonds between cells in the tissue. If the tissue is treated chemically and/or enzymatically, the cells of the tissue may be at least partly separated from each other, for example at the surface of the tissue, which may facilitate the embedding of the tissue in the hydrogel matrix, and may facilitate for example the survival, activity, proliferation, migration of substances, and/or integration of the tissue with the hydrogel. The chemically and/or enzymatically treated tissue sample(s) may be collected and/or concentrated, for example by using a centrifuge or filtration, to obtain tissue sample suitable for incorporating into the matrix. The tissue sample may also be untreated.

One embodiment comprises providing the patient-derived explant with a tumour-specific immune cell profile in a medium comprising one or more enzymes, such as a collagenase, and incubating the explant in the medium with the one or more enzymes, preferably to enzymatically digest bonds between cells in the explant, before embedding in the matrix comprising nanofibrillar cellulose hydrogel.

Tissue may be maintained as fragments or processed for generation of tissue slices of uniform sections, for example sections having a thickness of 200-300 µm, which are then cultured. The approach may not only facilitate drug diffusion but may also result in loss of some tissue integrity due to the increased set-up time.

However, in the processing of the sample it is important to maintain the corresponding microenvironment comprising the immune cells and optionally other bioactive substances. Especially fibroblasts, like the tumour-associated host fibroblasts (TAF), play a crucial role in immune regulation through TAF-derived extracellular matrix (ECM) components and modulators.

A cultured cell line represents a different material and is excluded from the present materials and methods.

The sample may be applied to the hydrogel as such, or it may be first processed, for example the dimensions of the tissue may be adjusted, for example cut, preferably to obtain a suitable thickness of the tissue which is better supported by the hydrogel environment.

The tissue to be used in preparing the patient derived explant for the *ex vivo* model should have suitable size or dimensions in order to be properly supported by the conditions provided by the matrix. The tissue or the tissue sample, which may be a processed tissue sample, may have the smallest average dimension of about 0.05 mm, 0.1 mm, about 0.2 mm, about 0.3 mm, about 0.4 mm, about 0.5 mm or about 1.0 mm, or more, or less, for example a smallest average dimension (thickness) of 3.0 mm or less, 2.0 mm or less, or 1.0 mm or less. The smallest average dimension may be in the range of 0.05-3 mm, 0.1-3 mm, such as 0.05-0.5 mm, 0.05-0.2 mm, 0.05-1.0 mm, 0.1-3.0 mm or 0.1-2.0 mm, for example 0.1-1.0 mm, and a longest average dimension may be 1.0 mm or more, 2.0 mm or more, 3.0 mm or more, 5.0 mm or more 7.0 mm or more or 10.0 mm or more, such as in the range of 1.0-20.0 mm, 1.0-10.0 mm, 1.0-5.0 mm, 3.0-20 mm, or 3.0-10.0 mm. It was surprisingly found out that tissue samples of such size ranges, especially including said smallest dimensions, could be maintained in the present model, which evidences that the matrix can be used to maintain materials containing a variety of substances ranging from tissue pieces to subcellular substances such as vesicles and macromolecules, also including, and/or ranging from or to, separate cells.

In one embodiment the model comprises a tissue sample, and/or the method comprises providing a tissue sample, having the smallest average dimension of 3.0 mm or less, 2.0 mm or less, 1.0 mm or less, or 0.5 mm or less, or a smallest average dimension in the range of 0.05-3.0 mm, such as 0.05-2.0 mm, 0.05-1.0 mm, 0.1-2 mm, 0.1-1.0 mm or 0.1-0.5 mm, or another range between any of the numerical values disclosed herein.

The sample may be a biopsy sample Examples of biopsies include needle biopsy, such as fine needle biopsy (aspiration) and core needle biopsy (core biopsy), excisional and incisional biopsy, wherein an entire tumour or a part of tumour is removed, endoscopic biopsy, laparoscopic, thoracoscopic, and mediastinoscopic biopsy, laparotomy and thoracotomy, skin biopsies, and sentinel lymph node mapping and biopsy. The dimensions of the sample may depend on the method of taking the sample, such as the method of taking biopsy.

The method comprises providing a matrix comprising nanofibrillar cellulose hydrogel, and combining the patient-derived explant with the matrix comprising nanofibrillar cellulose hydrogel to provide the *ex vivo* tumour immune microenvironment model.

The explant may be combined with the matrix by simply applying, embedding and/or mixing the matrix and/or the explant, if necessary, for example by gentle agitating, stirring, vortexing or otherwise mixing, optionally by using a spatula or the like manual mixing or manipulating means to properly embed the explant in the hydrogel. The applying may be carried out by using any suitable applying means, including a pipette, a syringe or a like, or the explant may be simply pressed into the hydrogel in some cases, for example by using tweezers, a spatula or the like. The explant may be completely covered or surrounded by the hydrogel matrix to confirm the maintenance, viability and activation of the explant in the matrix. In one example an amount of the hydrogel is applied onto the explant, for example to form a dome or the like covering form, to cover the explant at least partly. Combining the explant with the matrix may be carried out in a suitable container or compartment, such as in a well, for example in a well of a multiwell plate. In this way an array of *ex vivo* tumour immune microenvironment models can be prepared and provided. Suitable medium may be applied as well, such as one or more of mediums disclosed herein.

The concentration of the nanofibrillar cellulose has an impact to the efficiency of an immune cell type to survive or proliferate or being enriched. The concentration also has an impact to the cytokine profile in the model. In one example the concentration of the nanofibrillar cellulose in the hydrogel is in the range of 0.25-0.8% by weight, such as 0.3-0.7% by weight or 0.25-0.5% by weight, which represent a lower concentration range used in the present models. In one example the concentration of the nanofibrillar cellulose in the nanofibrillar cellulose hydrogel is in the range of 0.5-1.2% by weight, such as 0.6-1.2% by weight, 0.8-1.2% by weight, or 0.7-1.0% by weight, which represent a higher concentration range used in the present models. Such higher concentration may be used with specific type of NFC, for example anionic NFC, such as conjugated or functionalized anionic NFC. The higher concentration may be desired for certain types of immune cells, such as CD14+ monocytes, for example. The stiffness of the matrix also has an impact to said effects. The concentration may have an impact to the stiffness, but also features such as the type of NFC, and the modification and fibrillation degree and/or other features as discussed herein also impact to the stiffness. The present concentrations of NFC enable providing suitable permeability for different substances, which is required for the viability of the model components and for using the model.

The method may comprise culturing the tissue in the matrix comprising nanofibrillar cellulose hydrogel. The culturing may include maintaining the tissue and/or cells in the tissue, proliferating tissue and/or cells in the tissue, and/or maintaining or preserving the microenvironment. The maintaining or preserving may include maintaining, preserving, facilitating or enhancing the viability and/or activation of the tissue and/or cells in the tissue, including the immune cells, and/or the bioactive substances in the microenvironment, and/or preventing or suppressing clonal selection induced by *ex vivo* conditions, which may mean maintaining the diversity of the original tissue and/or cells in the tissue.

The culturing may be carried out at conditions facilitating the survival, viability, activity and/or proliferation of the tissue or cells in the tissue. The conditions may comprise a suitable culturing temperature, which may be in the range of 35-39°C, such as about 37°C and providing suitable culturing medium, which may contain one or more required nutrients, buffering agents, salts and optionally one or more bioactive agents or substances.

The present model is a 3D system or culture. 3D system or culture refers to a system or culture in the nanofibrillar cellulose, wherein the cells and/or tissue is/are permitted to grow and/or interact in all three dimensions in a matrix. The term "3D" is used to distinguish from 2D systems, which refer to systems or cultures in a membrane and/or as a layer. The NFC hydrogel matrix mimics the natural extracellular matrix structure and provides efficient transport of nutrients, gases and the like in 3D systems.

### The ex vivo model

The ex vivo model is a powerful scientific tool that can provide valuable insight into the function and behavior of complex biological systems. The obtained or provided *ex vivo* tumour immune microenvironment model may be used for examining the molecular mechanisms underlying the immune suppression of tumour microenvironment, and further developing relatively inexpensive platforms to specialized models which can be tailored to individual questions. The platforms are predictive of *in vivo* drug response and can rapidly generate drug response data.

The present models use patient-relevant material, and corresponding patient information and tumour diagnostic information are usually available. It is possible to study the surrounding normal tissue in parallel. In the model the tumours retain their proliferative capacity, tissue architecture and tumour-stroma interactions are maintained. Intra and inter-tumoural heterogeneity can be modelled. Agents targeting both tumour and stroma as well as immune cells can be assessed.

Multiple types of endpoint analysis can be applied, such as spatial and non-spatial. Multiple pharmacodynamic biomarkers can be profiled. The model allows correlation of drug response with tumour pathology, patient characteristics, genetics, drug distribution and/or biomarker expression.

The *ex vivo* tumour immune microenvironment model may be provided in one or more culture means, which may comprise one or more cell and/or tissue culture container(s), such as one or more culture plates, microwell plates, culture flasks or bottles, culture reactors or other bioreactors, and the like means, container or systems. Cell and/or tissue culture medium may be provided to the culture means, such as batchwise and/or continuously. The medium may be supplemented with one or more bioactive substances, such as cytokines. It was however found out that with the present matrix it was possible to promote the growth of immune cells without using additional biologically active compounds that are commonly used to affect proliferation and activation of the immune cells, or by using substantially less of such bioactive substances. Therefore in one example the medium is not supplemented with one or more bioactive substances, such as cytokines.

The method may comprise examining or monitoring one or more features from the *ex vivo* tumour immune microenvironment model, such as by taking samples and/or monitoring the model visually or optically, for example microscopically and/or by using computerized visual or optical means, and/or by using other monitoring means, such as detecting isotope or fluorescent labels and the like. In visual examination methods it is advantageous if the matrix is transparent and thus allows undisturbed visual examination of the components of the model, such as cancer tissue and cells, storma and/or immune cells, and reactions in the model. A highly fibrillar NFC, such as anionic NFC, especially oxidized NFC, provides such transparency and exhibits low turbidity, such as turbidity or 90 NTU or less, 60 NTU or less, or 40 NTU or less.

The *ex vivo* tumour immune microenvironment model may be used in tumour research methods, such as in cancer research methods, for example in drug screening methods. The cancer may be any suitable cancer type.

One example provides an *ex vivo* method for studying tumour tissue, such as cancer tissue, the method comprising
- providing the *ex vivo* tumour immune microenvironment model obtained from a patient of interest,
- providing one or more substances of interest to the model,
- detecting the reaction of the model to the one or more substances.

The method may be an *ex vivo* method for studying the effect of a substance of interest to tumour tissue obtained from a patient of interest, to stroma and/or to immune cells with or in the tumour tissue. The method may be carried out for example in wells of a multi-well plate.

The substance of interest may be for example a drug candidate molecule, a hormone, and/or any other suitable molecule or substance. For example the method may comprise providing an array, which may comprise two or more or a plurality, of different drug candidate molecules, or other molecules, and/or providing one or more, such as an array, of the models, and detecting the reaction of the model to each of the molecules, either separately and/or in combination of two or more such molecules. The reaction of the model may be for example a reaction relating to viability, activation and/or proliferation of the immune cells, stroma, the tumour tissue and/or tumour tissue cells, such as cancer tissue and/or cancer cells, such as decrease or increase in the proliferation, or viability of the tissue and/or tissue cells, or other reaction, such as binding of the substance to the tissue and/or cells, such as to a receptor, and optionally any reaction caused by such action.

In one embodiment the reaction of the model is change in viability, maintenance of viability, or an indication of a change in viability of the immune cells, such as one or more types of immune cells. The change in viability may be positive or negative. A positive change, *i.e.* wherein the immune cells show increased viability, increased activation and/or maintained viability, may indicate the suitability of the substance of interest for treating the tumour. In one embodiment the reaction of the model is inhibition of negative immune regulation. For example factors such as CTLA-4, PD-1 and the like can prevent T cells from killing cancer cells, and therefore it is possible to screen and identify candidate molecules suitable for suppressing or inhibiting the negative immune regulation.

One embodiment provides an *ex vivo* method for studying the effect of a substance of interest to tumour tissue obtained from a patient, such as cancer tissue, the method comprising
- providing the *ex vivo* tumour immune microenvironment model,
- providing one or more substances of interest to the model,
- detecting the reaction of the model to the one or more substances of interest, preferably wherein the reaction of the model is a change in viability, a change in activation, maintenance of viability, and/or an indication of a change in viability and/or a change in activation of tumour cells and/or the immune cells, such as wherein the change is increase. The method may be an *ex vivo* method for studying the effect of a substance of interest to immune profile of tumour tissue obtained from a patient, or to tumour immune microenvironment.

In one example the method comprises
- providing two or more of the *ex vivo* tumour immune microenvironment models, preferably each in a different well of a multi-well plate,
- providing two or more different substances of interests to the two or more *ex vivo* tumour tissue microenvironment models, each one of the models comprising one or more different substances,
- detecting the reactions of each model to the substances.

In one example the detecting the reaction of the model to the one or more substances may comprise a reaction indicating suitability of one or more of the provided substances as a drug for treating a disease or disorder of interest, such as cancer. In the case of cancer the reaction of the model may comprise increased or initiated activation of immune cells, decreased viability and/or proliferation of the cancer tissue and/or cells, such as death.

The reaction of the model to the one or more substances may be detected with any applicable detection means and/or methods, such as visual or optical means and/or methods, which refer to means and methods based on detecting light or images from the model. The light may comprise visible light, UV light and/or IR light. Such detection means may comprise microscopic means, such as light microscope and/or digital imaging means and associated software for analyzing the images or video and/or for detecting the reaction.

The detection means and/or methods may comprise labelling means and methods, such as antibodies specific for a marker, nucleic acid based methods such as RNA profiling, RNA sequencing, staining methods such as immunofluorescent staining, microscopical methods such as confocal microscopy, flow cytometry, cytokine profiling, radiological methods, and any combination thereof. For example features such as histological properties can be monitored and/or detected.

### Nanofibrillar cellulose

The starting material for forming the matrix may be nanofibrillar cellulose, also called as nanocellulose, which refers to isolated cellulose fibrils and/or fibril bundles separated from fibrous cellulose raw material. Nanofibrillar cellulose is based on a natural polymer that is abundant in nature. Nanofibrillar cellulose has a capability of forming viscous hydrogel in water. Nanofibrillar celluloses useful for the present purposes are not soluble in water, but they form a dispersion in water. Nanofibrillar cellulose production techniques may be based on disintegrating, *i.e.* fibrillating, fibrous raw material, such as grinding of aqueous dispersion of pulp fibers to obtain nanofibrillated cellulose. After the grinding or homogenization process, the obtained nanofibrillar cellulose material is a dilute viscoelastic hydrogel.

The obtained material usually exists at a relatively low concentration homogeneously distributed in water due to the disintegration conditions. The starting material may be an aqueous gel at a concentration of 0.2-10% (w/w), for example 0.2-5% (w/w). The nanofibrillar cellulose may be obtained directly from the disintegration of fibrous raw material, such as cellulose fibers, for example pulp. The nanofibrillar cellulose may be never-dried.

Because of its nanoscale structure nanofibrillar cellulose has unique properties which enable functionalities which cannot be provided by conventional non-nanofibrillar cellulose or for example synthetic fibers or fibrils. It is possible to prepare materials and products which exhibit different properties than conventional products or products using conventional cellulosic materials or other polymeric materials. However, because of the nanoscale structure nanofibrillar cellulose is also a challenging material. For example dewatering or handling of nanofibrillar cellulose may be difficult.

The nanofibrillar cellulose may be prepared from cellulose raw material of plant origin, or it may also be derived from certain bacterial fermentation processes. The nanofibrillar cellulose is preferably made of plant material. Nanofibrillar cellulose can be obtained from plants by mechanical disintegration of cellulose fibers to separate fibrils and/or fibril bundles. The raw material may be based on any plant material that contains cellulose. In one example the fibrils are obtained from non-parenchymal plant material. In such case the fibrils may be obtained from secondary cell walls. One abundant source of such cellulose fibrils is wood fibres. The nanofibrillar cellulose may be manufactured by disintegrating, such as homogenizing, wood-derived fibrous raw material, which may be pulp, such as chemical pulp. Cellulose fibers can be disintegrated to produce fibril bundles and/or even fibrils which have an average diameter of only some nanometers, to provide a dispersion of fibrils and/or fibril bundles in water. The fibrils originating from secondary cell walls are essentially crystalline with degree of crystallinity of at least 55%. Such fibrils may have different properties than fibrils originated from primary cell walls. In general in the cellulose sources from primary cell walls, such as sugar beet, potato tuber and banana rachis, the microfibrils are easier to liberate from the fibre matrix than fibrils from wood, and the disintegration requires less energy. However, these materials are still somewhat heterogeneous and comprise mainly large fibril bundles.

Non-wood material may be from agricultural residues, grasses or other plant substances such as straw, leaves, bark, seeds, hulls, flowers, vegetables or fruits from cotton, corn, wheat, oat, rye, barley, rice, flax, hemp, manila hemp, sisal hemp, jute, ramie, kenaf, bagasse, bamboo or reed. The cellulose raw material could be also derived from the cellulose-producing micro-organism. The microorganisms can be of the genus Acetobacter, Agrobacterium, Rhizobium, Pseudomonas or Alcaligenes, preferably of the genus Acetobacter and more preferably of the species Acetobacter xylinumor or Acetobacter pasteurianus.

It was found out that nanofibrillar cellulose obtained from plant cellulose, especially from wood cellulose, is preferable for life science, medical and/or scientific products described herein. The nanofibrillar cellulose obtained by fibrillating plant fibers, especially wood fibers, differs structurally from nanofibrillar cellulose obtained from microbes, and it has different properties. For example compared to bacterial cellulose, nanofibrillated wood cellulose is homogenous and more porous and loose material, which is advantageous in applications involving living cells. Bacterial cellulose is usually available as such without need for similar fibrillation as in plant cellulose, so the material is different also in this respect.

Nanofibrillar cellulose derived from plant material requires disintegrating cellulose fibers into fibrils and/or fibril bundles, which enables controlling the fibrillation process and the properties of the obtained fibrillated cellulose material. It is possible to modify the cellulose before the disintegration process and control the fibrillation type and efficiency for example by selecting a suitable device, suitable process conditions and time, and other related parameters, to obtain nanofibrillar cellulose with desired properties such as fibrillation degree, aspect ratio, modification type, stiffness and continuity.

Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. In one example the nanofibrillar cellulose is obtained from wood pulp. The nanofibrillar cellulose may be obtained from hardwood pulp. In one example the hardwood is birch. The nanofibrillar cellulose may be obtained from softwood pulp. In one example said wood pulp is chemical pulp.

Nanofibrillar cellulose, including the cellulose fibrils and/or fibril bundles, is characterized by a high aspect ratio (length/diameter). The length/diameter (L/D) ratio may be 5 or more, such as 10 or more, preferably 20 or more, 30 or more, 40 or more, or 50 or more. The average length of nanofibrillar cellulose (the median length of particles such as fibrils and/or fibril bundles) may exceed 1 µm, and in most cases it is 50 µm or less. If the elementary fibrils are not completely separated from each other, the entangled fibrils, such as fibril bundles, may have an average total length for example in the range of 1-100 µm, 1-50 µm, or 1-20 µm. This applies especially for native grades of fibrils which are not shortened or digested, for example chemically, enzymatically or mechanically. However, if the nanofibrillar material is highly fibrillated, the elementary fibrils may be completely or almost completely separated and the average fibril length is shorter, such as in the range of 1-10 µm or 1-5 µm. Strongly derivatized nanofibrillar cellulose may have a shorter average fibril length, such as in the range of 0.3-50 µm, such as 0.3-20 µm, for example 0.5-10 µm or 1-10 µm. Especially shortened fibrils, such as enzymatically and/or chemically digested fibrils, or mechanically treated material, may have an average fibril length of less than 1 µm, such as 0.1-1 µm, 0.2-0.8 µm or 0.4-0.6 µm. If high aspect ratio is desired, it is not desired to shorten the fibril length so it may not be desired to use chemically or enzymatically hydrolysed or digested cellulose, or such highly mechanically fibrillated material where the fibrils are already shortened.

The fibril length and/or diameter may be determined with several techniques, such as by microscopy, especially electron microscopy. Fibril thickness and/or width distribution may be measured by image analysis of microscope images, such as images from a scanning electron microscope (SEM), for example field emission scanning electron microscope (FE-SEM), a transmission electron microscope (TEM), for example a cryogenic transmission electron microscope (CRYO-TEM), or an atomic force microscope (AFM). In general AFM and TEM, especially CRYO-TEM, suit best for nanofibrillar cellulose grades with narrow fibril diameter distribution. From Cryo-TEM images, also the bundled structure can be seen. A suitable imaging software may be used.

The average diameter (width) of nanofibrillar cellulose is less than 1 µm, or 500 nm or less, such as in the range of 1-500 nm, but preferably 200 nm or less, even 100 nm or less, or 50 nm or less, such as in the range of 1-200 nm, 1-100 nm, or 1-50 nm, even 1-20 or 5-30 nm for highly fibrillated and/or chemically modified cellulose. The diameters disclosed herein refer to fibrils and/or fibril bundles. The smallest fibrils are in the scale of elementary fibrils, the average diameter being typically in the range of 4-12 nm. The dimensions and size distribution of the fibrils depend on the refining method and efficiency, and on possible modification type and degree. In case of highly refined native nanofibrillar cellulose, the average fibril and/or fibril bundle diameter may be in the range of 2-200 nm or 2-100 nm, for example in the range of 10-50 nm. In case of highly refined chemically modified nanofibrillar cellulose, the average fibril and/or fibril bundle diameter is usually lower.

The average lengths and/or diameters disclosed herein may be number-average lengths and/or diameters. The term "fibrils" as used herein also includes fibril bundles, where applicable. The mechanically disintegrated cellulosic material may contain at least a small amount of cellulose which is not fully separated into elemental fibrils but is still in the form of fibril bundles.

It is also not desired that the fibrils are hornified, in which case irreversible agglomeration of fibrils deteriorate the quality of the nanofibrillar cellulose and lowers the aspect ratio, and consequently results in the complete loss of its beneficial properties. Hornification can occur during post-processing, such as drying of aqueous NFC suspensions, and it can be explained with the formation of a large number of hydrogen bonds between the hydroxyl groups of adjacent nanofibrils. A lowered aspect ratio, or hornified material, can be usually detected as decreased viscosity of NFC dispersion, and/or even microscopically.

In general nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose obtained from plants, especially wood, may also contain small amounts of other plant components, especially wood components, such as hemicellulose or lignin. The amount may be dependent on the plant source, and/or on the type of raw material. For example chemical pulp may contain less or substantially none of other plant components.

In one example cellulose nanomaterials can be divided into categories according to TAPPI W13021 (2014), which provides standard terms for cellulose nanomaterials. Not all of these materials are nanofibrillar cellulose, and therefore cannot exhibit similar properties. Two main categories are "Nano objects" and "Nanostructured materials". Nanostructured materials include "Cellulose microcrystals" (sometimes called as CMC) having a diameter of 10-15 µm and length:diameter ratio (L/D) <2, and "Cellulose microfibrils" having a diameter (width) of 10-100 nm and a length of 0.5-50 µm. Nano objects include "Cellulose nanofibers", which can be divided into "Cellulose nanocrystals" (CNC) having a diameter of 3-10 nm and L/D >5, and "Cellulose nanofibrils" (CNF or NFC), having a diameter of 5-30 nm and L/D >50. In one example the nanofibrillar cellulose has a number-average diameter in the range of 5-30 nm and a length/diameter (L/D) ratio of 50 or more.

Nanocrystalline cellulose is different material from nanofibrillar cellulose, and has different properties and behaviour. Nanocrystalline cellulose is produced from cellulose by acid hydrolysis, which removes the amorphous region to obtain the crystalline region of cellulose. The fibril length is substantially shortened. Nanofibrillar cellulose as used herein is also not meant to include cellulose nanowhiskers or other rod-like particles, or the fines formed in conventional cellulose pulping processes, for example as small amounts on the surface of cellulose fibers.

Different grades of nanofibrillar cellulose can be also categorized based on three main properties: (i) size distribution, length and/or diameter (ii) chemical composition, and (iii) rheological properties. To fully describe a grade, two or more properties may be used in parallel. Examples of different grades include native (chemically and/or enzymatically unmodified) NFC, oxidized NFC (high viscosity), oxidized NFC (low viscosity), carboxymethylated NFC and cationized NFC. Within these main grades, also sub-grades exist, for example: extremely well fibrillated vs. moderately fibrillated, high degree of substitution vs. low degree of substitution, low viscosity vs. high viscosity etc. The fibrillation technique and the chemical pre-modification both have an influence on the properties of fibrillar cellulose, such as on fibril size and fibril size distribution. Typically, non-ionic grades have wider average fibril and/or fibril bundle diameter (for example in the range of 10-100 nm, or 10-50 nm) while the chemically modified grades are a lot thinner (for example in the range of 2-20 nm). Distribution is also narrower for the modified grades. Certain modifications, especially TEMPO-oxidation, yield shorter fibrils. However the fibril size and distribution and not the only properties that can be controlled during manufacturing process, as it is also possible to control the rheological properties of the obtained material. The main properties and subgrades thereof are not necessarily directly proportional to each other. For example fibrillar celluloses having the same diameter may not exhibit the same rheological properties, and/or other properties such as storage modulus, permeability and the like discussed herein.

Also pre-processing and/or post-processing of the cellulose may have an influence on the properties of the cellulose. It may be for example desired to include one or more non-fibrillating pre-processing or post-processing steps to control the continuity of the material and/or to provide material suitable for certain disintegration device. It is also desired to avoid damaging the obtained nanofibrillar cellulose in post-processing steps or during handling and storing, for example to avoid hornification.

In an aqueous environment, a dispersion of cellulose nanofibrils forms a viscoelastic hydrogel network. The gel is formed already at low concentrations of for example 0.05-0.2% (w/w) by dispersed and hydrated entangled fibrils. The viscoelasticity of the NFC hydrogel can be characterized for example with dynamic oscillatory rheological measurements, and also these properties differ significantly from those of conventional materials.

The nanofibrillar cellulose hydrogels or dispersions exhibit characteristic rheological properties and non-Newtonian behaviour, *i.e.* the nanofibrillar cellulose dispersions represent non-Newtonian fluids. For example they are shear-thinning or pseudoplastic materials, which may be considered as a special case of thixotropic behavior, which means that their viscosity depends on the speed or force by which the material is deformed. When measuring the viscosity in a rotational rheometer, the shear-thinning behavior is seen as a decrease in viscosity with increasing shear rate. The hydrogels show plastic behavior, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity is seen after exceeding the critical shear stress. The zero shear viscosity and the yield stress are the most important rheological parameters to describe the suspending power of the materials. These two parameters separate the different grades quite clearly and thus enable classification of the grades. The zero shear viscosity and the yield stress can be also used to distinguish nanofibrillar celluloses from Newtonian materials, such as conventional fibrous cellulosic materials, which do not exhibit similar dramatic decrease in viscosity when the viscosity is plotted as function of applied shear stress, but rather show a continuous substantially linear curve.

As used herein, the term "fibrillation" generally refers to disintegrating fiber material mechanically by work applied to the particles, where cellulose fibrils and/or fibril bundles are detached from the fibers or fiber fragments and fibrillar cellulose is obtained. The work may be based on various effects, like grinding, crushing or shearing, or a combination of these, or another corresponding action that reduces the particle size. The expressions "disintegration" or "disintegration treatment" may be used interchangeably with "fibrillation". The fibrillating treatment requires significantly more energy compared to conventional refining of cellulose in pulping, and the devices and/or settings of the devices may be different.

The fiber material dispersion that is subjected to fibrillation is a mixture of fiber material and water, also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

The dimensions of the fibrils and/or fibril bundles, as well as other properties of the nanofibrillar cellulose, are dependent for example on the raw material, the disintegration method and number of disintegration runs. Mechanical disintegration of the cellulose raw material may be carried out with any suitable equipment such as a refiner, grinder, disperser, homogenizer, colloider, friction grinder, pin mill, rotor-rotor disperser, ultrasound sonicator, fluidizer such as microfluidizer, macrofluidizer or fluidizer-type homogenizer, or a combination thereof. The disintegration treatment is performed at conditions wherein water is sufficiently present to prevent the formation of bonds between the fibers and/or fibrils, which could result in agglomeration of the fibers and/or fibrils

In one example the disintegration is carried out by using a disperser having at least one rotor, blade or similar moving mechanical member, such as a rotor-rotor disperser, which has at least two rotors. One example of a rotor-rotor disperser is an Atrex device. Another example of a device suitable for disintegrating is a pin mill, such as a multi-peripheral pin mill. In one example the disintegrating is carried out by using a homogenizer. A homogenizer can also be used for homogenizing cellulosic material without fibrillating, depending on said adjustment and process conditions.

The hydrogel to be used in the present applications is preferably homogenous. Therefore the preparation method may include homogenizing a hydrogel comprising nanofibrillar cellulose, preferably with a homogenizing device such as ones described herein. With this preferably non-fibrillating homogenizing step it is possible to remove areas of discontinuity from the gel. Especially it is possible to control the properties of wood cellulose with such treatment. A homogenous gel having better properties for the present applications is obtained. The non-fibrillating homogenizing step may be carried out after one or more fibrillating steps and/or after one or more concentrating steps, which may cause the fibrils and/or fibril bundles to aggregate thus having an impact to the continuity and homogeneity of the hydrogel. The hydrogel may be further sterilized, for example by using heat, such as by autoclaving, and/or by using radiation, and/or by adding sterilizing agents, such as antimicrobials.

One way to characterize the nanofibrillar cellulose is to use the viscosity of an aqueous solution or dispersion containing said nanofibrillar cellulose. The viscosity may be for example Brookfield viscosity or zero shear viscosity. The specific viscosity, as described herein, can be used to distinguish nanofibrillar cellulose from non-nanofibrillar cellulose, and/or to define the fibrillation degree.

The nanofibrillar cellulose may also be characterized by the average diameter (or width), or by the average diameter together with the viscosity, such as zero shear viscosity.

Degree of fibrillation can be evaluated by using fiber analysis where number of larger, only partially fibrillated, entities are evaluated. For example, in the case of derivatized nanofibrillar cellulose the number of those particles per mg of dry sample may be in the range of 0-10000, such as in the range of 0-5000, for example in the range of 0-1000. However, in non-derivatized NFC the number of non-fibrillated particles/mg is typically somewhat higher in the range of 0-20000, such as in the range of 0-10000, for example in the range of 0-5000. The fiber analysis may be carried out using Fiberlab method.

The mechanical stiffness of the nanofibrillar cellulose hydrogels can be evaluated from viscoelastic measurements of the gels. "Mechanical stiffness" refers to the resistance of an elastic body to deflection or deformation by an applied force. Storage modulus can be used for characterizing the stiffness, and also the gelling state of the nanofibrillar cellulose hydrogel. Derivatized NFC may build up stiffer hydrogels, but extensive fibrillation of these grades may lead also to lower storage modulus. The stiffness has an impact to the properties of the hydrogel, not only the mechanical properties but also to the behaviour of the embedded cells and/or tissue together with the microenvironment. By selecting a specific stiffness such as disclosed herein it is possible to provide a suitable pressure for the tissue and cells and to maintain the whole tissue microenvironment in a desired form and active in the hydrogel.

The storage modulus may be measured at the concentration of the hydrogel and at 37±1°C, wherein it reflects the stiffness of the hydrogel during the use such as tissue or cell culture and/or other procedures disclosed herein. In general the nanofibrillar cellulose, when dispersed in water at 0.25-1.2% by weight, such as 0.3-1.1% by weight, of the nanofibrillar cellulose hydrogel, provides a storage modulus in the range of 1-1000 Pa at pH 7 and at 37±1°C determined by a rotational rheometer.

In one embodiment the nanofibrillar cellulose, when dispersed in water at 0.25-1.2% by weight, such as 0.3-1.1% by weight, of the nanofibrillar cellulose hydrogel, provides a storage modulus in the range of 1-500 Pa, such as in the range of 2-100 Pa, for example 2-50 Pa, at pH 7 and at 37±1°C determined by a rotational rheometer.

It is possible to control the properties, such as storage modulus, of the obtained nanofibrillar cellulose hydrogel during the manufacturing process by selecting a suitable device and controlling the fibrillation process and parameters.

In general the properties of the NFC matrix, such as modification, concentration, stiffness, dimensions and other properties discussed herein may be determined from the NFC matrix hydrogel without the tissue or the sample comprising the tissue, such as by taking a sample of the hydrogel only and determining the properties from the sample.

A rheometer viscosity of the nanofibrillar cellulose dispersion may be measured according to one example at 22°C with a stress controlled rotational rheometer (AR-G2, TA Instruments, UK) equipped with a narrow gap vane geometry (diameter 28 mm, length 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s⁻¹ is exceeded. This method may be used for determining the zero-shear viscosity.

In another example rheological measurements of the hydrogel samples were carried out with a stress controlled rotational rheometer (AR-G2, TA instruments, UK) equipped with 20 mm plate geometry. After loading the samples to the rheometer, 1 mm gap, without dilution, they were allowed to settle for 5 min before the measurement was started. The stress sweep viscosity was measured with gradually increasing shear stress in a range of 0.001-100 Pa at the frequency 10 rad/s, strain 2%, at 22±1°C. Storage modulus, loss modulus and yield stress/fracture strength can be determined.

In one example the nanofibrillar cellulose, for example provided as a starting material in the method, when dispersed in water, provides a zero shear viscosity ("plateau" of constant viscosity at small shearing stresses) in the range of 100-50000 Pa·s, or in the range of 1000-50000 Pa·s, In most cases useful for the present purposes the zero shear viscosity is in the range of 3000-50000 Pa s, such as in the range of 5000-30000 Pa s or 3000-20000 Pa s and a yield stress (shear stress where the shear thinning begins) in the range of 1-50 Pa, such as in the range of 2-15 Pa or 3-15 Pa, determined by rotational rheometer at a consistency of 0.5% (w/w) by weight in aqueous medium at 22±1°C. Such nanofibrillar cellulose may also have a number-average fibril diameter of 200 nm or less, such as in the range of 1-200 nm, 1-100 nm, 1-50 nm or 1-20 nm. Such material is fibrillated into such degree and has such properties that especially facilitates the applications discussed herein. Particularly the zero shear viscosity in the range of 5000-30000 Pa s and a yield stress in the range of 3-15 Pa are especially suitable for the present applications.

The rheological properties, as well as other properties, such as turbidity, as disclosed and discussed herein, are usually determined at standard conditions, including a standard consistency, for example 0.5 wt% or 0.8 wt% consistency or concentration in water, such as in pure water, or in aqueous medium. The aqueous medium may be water, such as pure or purified water, or an aqueous medium obtained by adding said water. The nanofibrillar cellulose, such as a sample taken from the nanofibrillar cellulose, may be therefore dispersed in water to obtain the desired standard consistency for determining one or more rheological property or other property, which is denoted herein by the expression "when dispersed in water". Dispersing may be carried out by using a suitable mechanical disintegration treatment which causes homogenous dispersion of the NFC into the aqueous solution without further fibrillating the cellulose. A homogenous dispersion of NFC is obtained. In case the nanofibrillar cellulose has a lower consistency than the desired measuring consistency, it can be concentrated and optionally further dispersed to obtain the desired consistency and homogeneity.

One way to characterize nanofibrillar cellulose is to define turbidity, or both the viscosity and turbidity. Low turbidity refers to small size of the fibrils, such as small diameter, as small fibrils scatter light poorly. In general as the fibrillation degree increases, the viscosity increases and at the same time the turbidity decreases. This happens, however, until a certain point. When the fibrillation is further continued, the fibrils finally begin to break and shorten, and therefore cannot form a strong network anymore. Therefore, after this point, both the turbidity and the viscosity begin to decrease.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample. The turbidity may be measured according to ISO 7027.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1%. HACH P2100 Turbidometer with a 50 ml measuring vessel is used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

In one example the turbidity of chemically anionically modified nanofibrillar cellulose is 90 NTU or less, 60 NTU or less, or 40 NTU or less, for example from 3 to 90 NTU, such as from 5 to 60, for example 8-40, measured by nephelometry at a consistency of 0.1% (w/w) in aqueous medium. In one example the turbidity of native nanofibrillar may be even over 200 NTU, for example from 10 to 220 NTU, such as from 20 to 200, for example 50-200 measured by nephelometry at 20°C±1°C a consistency of 0.1% (w/w) in aqueous medium . To characterize the nanofibrillar cellulose these ranges may be combined with the viscosity ranges of the nanofibrillar cellulose, such as zero shear viscosity, storage modulus and/or yield stress.

Nanofibrillar cellulose may be or comprise non-modified nanofibrillar cellulose. The drainage of non-modified nanofibrillar cellulose is significantly faster than for example anionic grade. Non-modified nanofibrillar cellulose generally has a Brookfield viscosity in the range of 2000-10000 mPa·s, measured at 20°C±1°C, at a consistency of 0.8% (w/w) and at 10 rpm.

The disintegrated fibrous cellulosic raw material may be modified fibrous raw material. Modified fibrous raw material means raw material where the fibers are affected by the treatment so that cellulose nanofibrils are more easily detachable from the fibers. The modification is usually performed to fibrous cellulosic raw material which exists as a suspension in a liquid, i.e. pulp.

The modification treatment to the fibers may be chemical, enzymatic and/or physical. In chemical modification the chemical structure of cellulose molecule is changed by chemical reaction ("derivatization" of cellulose), preferably so that the length of the cellulose molecule is not affected but functional groups are added to β-D-glucopyranose units of the polymer. The chemical modification of cellulose takes place at a certain conversion degree, which is dependent on the dosage of reactants and the reaction conditions, and as a rule it is not complete so that the cellulose will stay in solid form as fibrils and does not dissolve in water. In physical modification anionic, cationic, or non-ionic substances or any combination of these are physically adsorbed on cellulose surface. The modification of the starting material, the fibrous cellulose, usually remains in the obtained nanofibrillar cellulose, for example chemically modified cellulose fibers can be used to obtain chemically modified nanofibrillar cellulose.

The cellulose in the fibers may be especially ionically charged after the modification. The ionic charge of the cellulose weakens the internal bonds of the fibers and will later facilitate the disintegration to nanofibrillar cellulose. The ionic charge may be achieved by chemical or physical modification of the cellulose. The fibers may have higher anionic or cationic charge after the modification compared with the starting raw material. Most commonly used chemical modification methods for making an anionic charge are oxidation, where hydroxyl groups are oxidized to aldehydes and carboxyl groups, sulphonization and carboxymethylation. Chemical modifications introducing groups, such as carboxyl groups, which may take part in forming a covalent bond between the nanofibrillar cellulose and the bioactive molecule, may be desired. A cationic charge in turn may be created chemically by cationization by attaching a cationic group to the cellulose, such as quaternary ammonium group.

Nanofibrillar cellulose may comprise chemically modified nanofibrillar cellulose, such as anionically modified nanofibrillar cellulose or cationically modified nanofibrillar cellulose. In one example the nanofibrillar cellulose is anionically modified nanofibrillar cellulose. In one example the anionically modified nanofibrillar cellulose is oxidized nanofibrillar cellulose. In one example the anionically modified nanofibrillar cellulose is sulphonized nanofibrillar cellulose. In one example the anionically modified nanofibrillar cellulose is carboxymethylated nanofibrillar cellulose. The material obtained with the anionical modification of cellulose may be called anionic cellulose, which refers to material wherein the amount or proportion of anionic groups, such as carboxylic groups, is increased by the modification, when compared to a non-modified material. It is also possible to introduce other anionic groups to the cellulose, instead or in addition to carboxylic groups, such as phosphate groups or sulphate groups. The content of these groups may be in the same ranges as is disclosed for carboxylic acid herein. The chemical modification or derivatization degree shall be in a range, which facilitates the fibrillability of the cellulose and provides advantageous effects to the properties of the obtained nanofibrillar cellulose, but which does not make the nanofibrillar cellulose soluble, wherein the properties thereof would change significantly.

In the chemical derivatization process a desired degree of substitution of the cellulose can be obtained. This may be carried out by controlling the chemical derivatization process, such as by selecting suitable raw material(s), reaction conditions, equipment, reaction time, reagents and/or the like properties. The oxidized NFC may contain also aldehyde functional groups, typically in the range of 10-250 micromol/g cellulose such as pulp, such as 50-250 micromol/g. Derivatization via carboxymethylation is typically conducted for cellulose pulp to ds levels in the range of 0.05-0.3 micromol/g, preferably 0.08-0.25 micromol/g, most preferably 0.10-0.2 micromol/g prior to fibrillation. If the derivatization is conducted by cationization, the DS levels are typically in the range of 0.05-0.4 micromol/g, preferably 0.15-0.3 micromol/g.

The cellulose may be oxidized. In the oxidation of cellulose, the primary hydroxyl groups of cellulose may be oxidized catalytically by a heterocyclic nitroxyl compound, such as through N-oxyl mediated catalytic oxidation, for example 2,2,6,6-tetramethylpiperidinyl-1-oxy free radical, generally called "TEMPO". The primary hydroxyl groups (C6-hydroxyl groups) of the cellulosic β-D-glucopyranose units are selectively oxidized to carboxylic groups. Some aldehyde groups are also formed from the primary hydroxyl groups. Regarding the finding that low degree of oxidation does not allow efficient enough fibrillation and higher degree of oxidation inflicts degradation of cellulose after mechanical disruptive treatment, the cellulose may be oxidized to a level having a carboxylic acid content in the oxidized cellulose in the range of 0.5-2.0 mmol COOH/g pulp (or mmol/g cellulose), 0.6-1.4 mmol COOH/ g pulp, or 0.8-1.2 mmol COOH / g pulp, preferably to 1.0-1.2 mmol COOH/ g pulp, determined by conductometric titration. For TEMPO or N-oxyl mediated oxidation is typically conducted to charge values in the range of 0.3-1.5 mmol COOH/ g pulp, preferably 0.6-1.2 mmol COOH/ g pulp, most preferably 0.7-1.1 mmol COOH/ g pulp.

It was found out that chemically anionically modified nanofibrillar cellulose, such as oxidized cellulose was preferred for certain uses. In one embodiment the nanofibrillar cellulose comprises, or is, oxidized nanofibrillar cellulose, such as TEMPO oxidized nanofibrillar cellulose.

Whenever the catalyst "TEMPO" is mentioned in this disclosure, it is evident that all measures and operations where "TEMPO" is involved apply equally and analogously to any derivative of TEMPO or any heterocyclic nitroxyl radical capable of catalyzing selectively the oxidation of the hydroxyl groups of C6 carbon in cellulose.

When the fibers of oxidized cellulose so obtained are disintegrated in water, they give stable transparent dispersion of individualized cellulose fibrils, which may be, for example, of 3-5 nm in width in average. With oxidized pulp as the starting medium, it is possible to obtain nanofibrillar cellulose where Brookfield viscosity measured at a consistency of 0.8% (w/w) is at least 10000 mP·as, for example in the range of 10000-30000 mPa·s, measured at 20°C±1°C at a consistency of 0.8% (w/w) and at 10 rpm.

Auxiliary agents for enhancing the manufacturing process or improving or adjusting the properties of the NFC or products formed from the NFC may be included in the nanofibrillar cellulose dispersion. Such auxiliary agents may be soluble in the liquid phase of the dispersion, they may form an emulsion or they may be solid. Auxiliary agents may be added already during the manufacturing of the nanofibrillar cellulose dispersion to the raw material or they may be added to a formed nanofibrillar cellulose dispersion or gel.

The present application discloses use of nanofibrillar cellulose for preparing the hydrogels. The nanofibrillar cellulose may be any suitable nanofibrillar cellulose disclosed herein. The present application also discloses use of nanofibrillar cellulose for any of the method and purposes disclosed herein, such as examination methods, cancer research, drug research and/or discovery, biomarker studies, signalling studies, diagnostic methods and the like.

The nanofibrillar cellulose may also be the only fibrillar, fibrous, polymeric, cellulosic and/or organic substance in the hydrogel, so in such case there may be no other matrix and/or reinforcing material in addition to nanofibrillar cellulose. The hydrogel may not contain other reinforcing and/or matrix-like materials or structures, for example synthetic, semi-synthetic or natural polymers or other compounds, such as collagens, proteins or peptides or other biological polymers, or plastic polymers.

The nanofibrillar cellulose may be functionalized with one or more functional molecules, such as cytokines, extracellular matrix proteins such as laminin or fibronectin, pharmaceutical molecules and/or antibodies. The functional molecules may be bioactive molecules, and they may be originated from a variety of sources, for example they may be obtained recombinantly and/or isolated from an organism. The functional molecule may contain a covalently attached (conjugated) binding molecule, which enables the functional molecule to non-covalently bind to a receiving molecule conjugated to the nanofibrillar cellulose. Examples of such conjugatable binding molecules include biotin and avidin.

In one embodiment the nanofibrillar cellulose comprises avidin-conjugated nanofibrillar cellulose. Avidin enables functionalizing the nanofibrillar cellulose with biotinylated molecules, which can be used to functionalize the nanofibrillar cellulose with one or more biotinylated functional molecules. Alternatives to avidin include streptavidin and neutravidin. The non-covalent bond between avidins and biotin has very high binding affinity, which makes is especially suitable for the present purposes. Biotinylation can be carried out chemically or enzymatically.

When the nanofibrillar cellulose is conjugated with avidin, it may be necessary to add or include (unconjugated) biotin in the cell culture medium to block free avidins in the hydrogel. This can prevent or decrease possible adverse effects caused by free avidins, such as inflammatory reactions.

In one embodiment the nanofibrillar cellulose is functionalized with one or more cytokines. Cytokines were found to enable and/or facilitate viability, culturing, maintaining and activating the tissue and the cells, especially immune cells, in the conditions of the present *ex vivo* model system. It was found out the cytokines support immune cell survival and maintenance of tumour-infiltrating lymphocytes. The cytokine may comprise a biotinylated cytokine, which can be any suitable cytokine, or a cytokine conjugated to any other suitable binding molecule allowing the functionalization of the NFC matrix. Cytokines represent a broad variety of small proteins or peptides, usually of about 5-25 kDa, which have a role in cell signalling. Examples of cytokines include chemokines, interferons, interleukins, lymphokines, and tumour necrosis factors, but generally not hormones or growth factors. One example of a cytokine is interleukine (IL), such as interleukine 2 (IL-2). In one embodiment the nanofibrillar cellulose is functionalized with biotinylated IL-2.

The concentration of the nanofibrillar cellulose in the nanofibrillar cellulose hydrogel is in the range of 0.25-1.2% by weight. In most cases the concentration may be in the range of 0.3-1.1% by weight.

### Examples

The percentages refer to weight percentages (by weight, wt%, w/w) unless otherwise indicated. Nanofibrillar celluloses obtained from wood cellulose were selected to the tests, including chemically non-modified NFC (NFC), transparent oxidized anionic NFC (ANFC) and avidin-conjugated transparent oxidized anionic NFC (ANFC-A). The oxidized anionic NFC comprised fibrils and/or fibril bundles having a number-average diameter below 50 nm or even below 25 nm, determined from electron microscopy images.

### Optimization of NFC for PDECs:

NFC concentrations for PDECs were optimized. Following matrix concentrations were found to support PDEC survival for 7 days in culture:
NFC 0.3% & 1.0%
ANFC 0.3% & 0.7%
ANFC-A 0.3% & 0.7%

ANFC-A was functionalized with biotinylated IL-2. Functionalized matrix could also support PDEC survival for 7 days in culture.

### Optimization of NFC for PDEC-TIME:

ANFC-A was found to cause an inflammatory reaction, which could be prevented by additional supplementation of biotin in the growth media. Patient specific immune cell profiles were retained in the nanofibrillar celluloses in general. ANFC-A and ANFC performed better than NFC in tumour resident immune cell preservation. TNFα and IL-1β production was higher in the more concentrated matrices. IL-6 and TGFβ production increased overtime in the lower concentrated matrices. The optimalization was successful by revealing which of the tested matrices and concentrations suited the best for different immune cell types.

Aforementioned matrix concentrations were also found to support PBMC immune cell survival. However ANFC-A was found to cause an inflammatory reaction, which could be prevented by additional supplementation of biotin in the growth media. NFCs gave a growth benefit for the PBMC over the conventional floating method without the need of IL-2 supplementation. Of the nanocelluloses, T-cells were more prevalent in ANFC-A and ANFC in high polymer concentrations. NK/NKT cells and CD14- monocytes responded most positively to IL-2 functionalization. Monocytes respond differently to different polymer concentrations.

### Research questions background

PDECs survive and grow well in NFC matrices in standard 7-day culture conditions. However, it was not known, how accurately the viability and proliferation status of PDECs grown in nanofibrillar cellulose mirrors the original tumour in long-term cultures. Defining what is the degree of reciprocity of the original tumour and PDECs in NFCs is essential for all experiments planned in the current research and therefore, the metrics pertinent to proliferation and viability will be evaluated first. The proliferation will be compared to the original tumour sample.

### Validating the suitability of cellulose-based matrices as scaffolds for "patient-derived breast cancer explant cultures with tumour immune microenvironment" (TIME-PDEC)

### Suitability of NFC for PDECs

The increase in the matrix concentration was to prevent the loss of explants during the immunofluorescent staining. Proper difference between low and high concentration was, however, maintained. ANFC-A was agreed to be functionalized with introduction of biotinylated interleukin-2 (IL-2), to promote immune cell survival in NFC. IL-2 is expected to induce T-cell proliferation and support immune cell survival in tumour microenvironment, this is why it was previously also called a T-cell growth factor. The explant viability metrics were compared to the results of the uncultured, day 0 samples, which represent the original tumour sample and will thus reveal any change in the explant viability during the ex vivo culture.

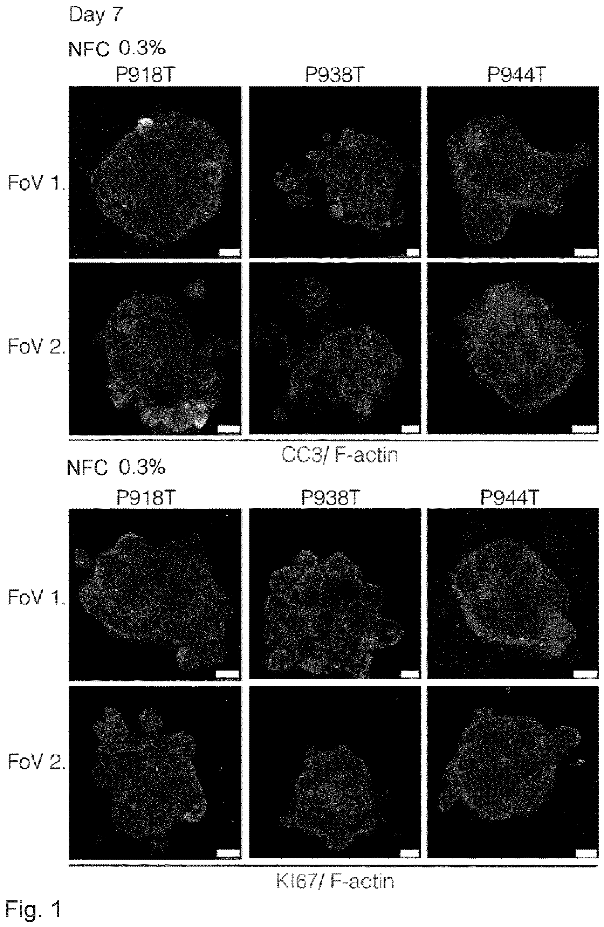

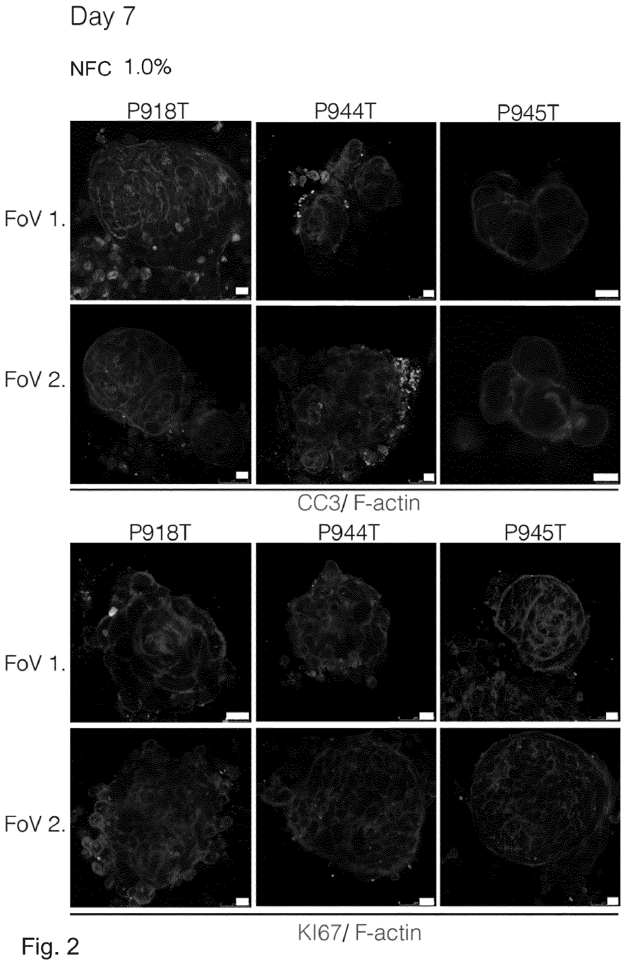

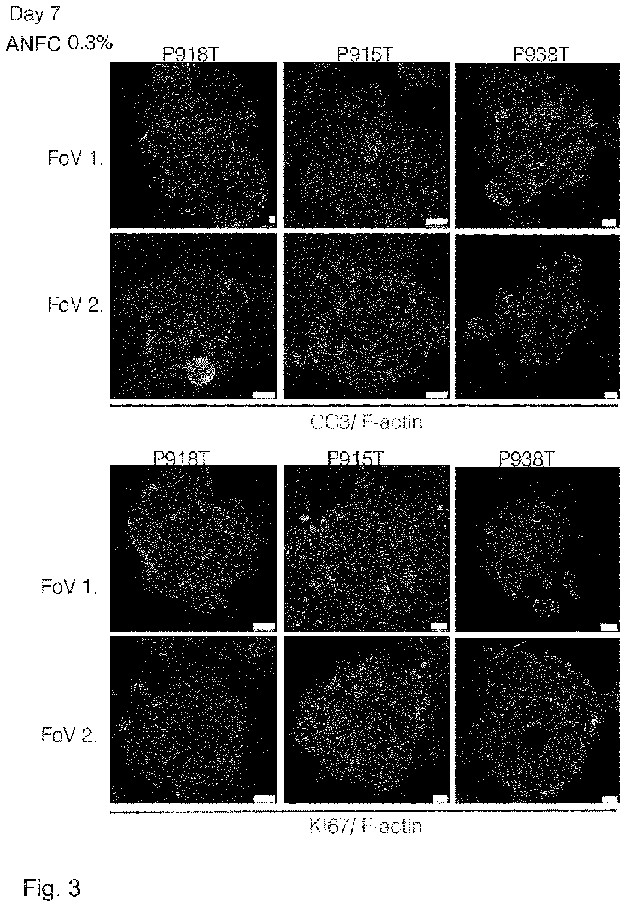

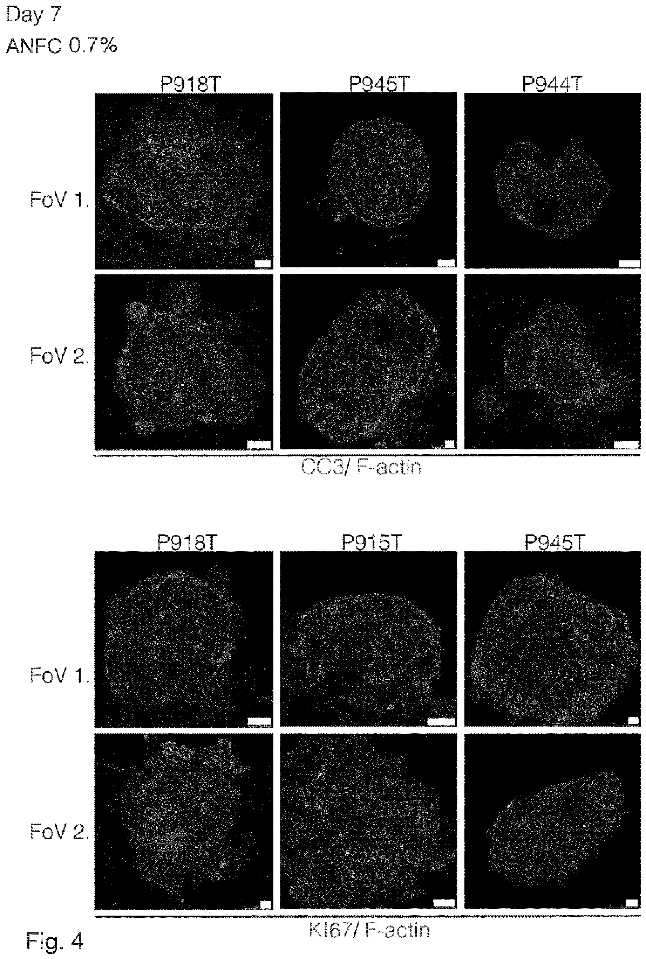

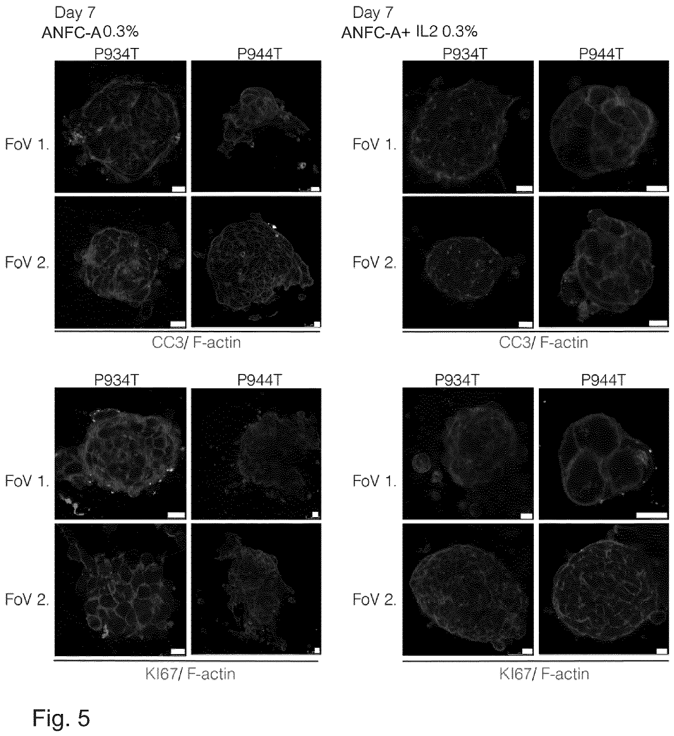

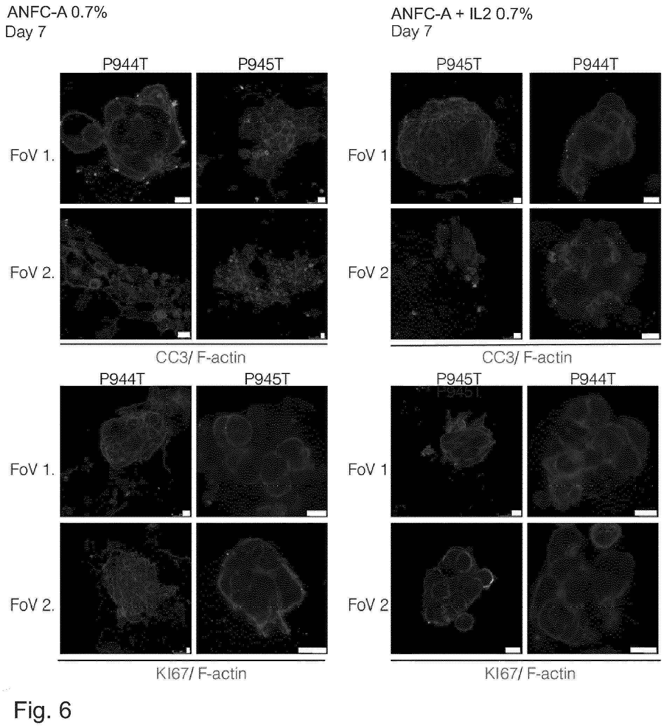

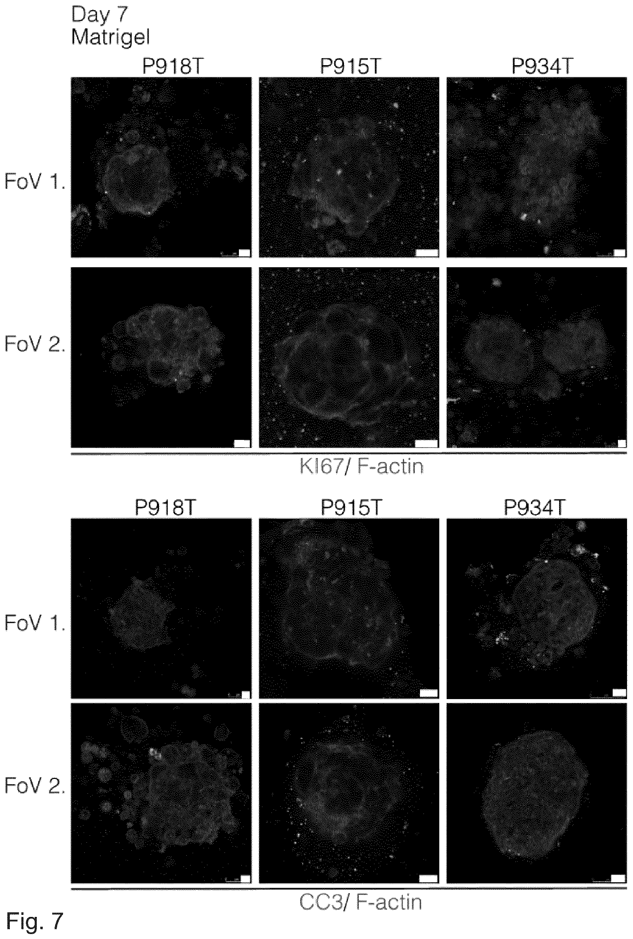

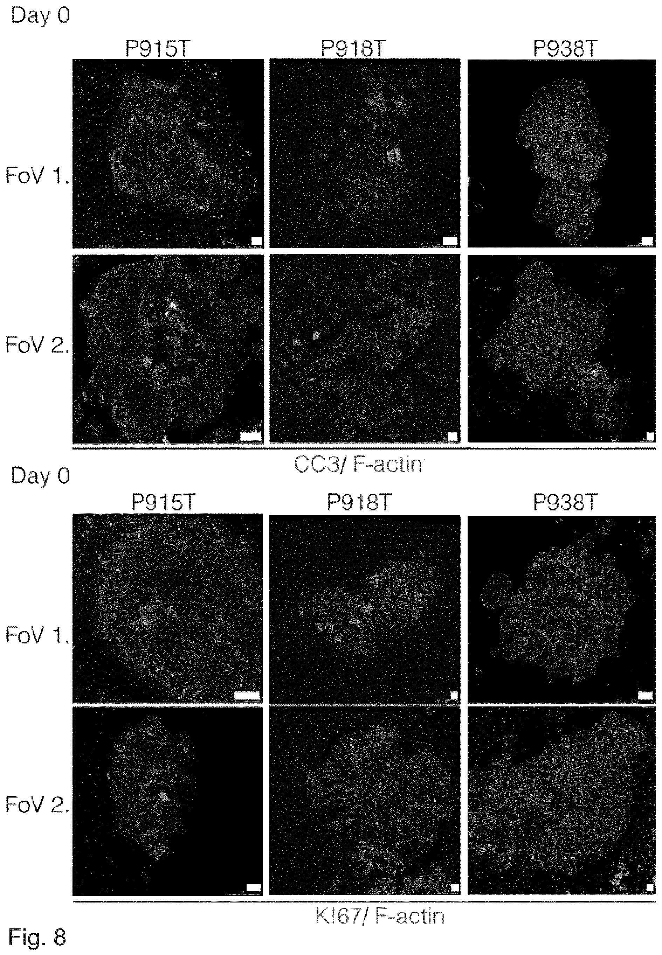

Matrigel cultures, which is also known to preserve the explant viability, were used as a control for explant *ex vivo* growth. Each tested NFC matrix, despite of their concentration, was found to preserve original explant viability. The explants were compared to their uncultured counterparts, which represent the original tumour and to the Matrigel cultures. The explants preserved their 3D structures as observed with cell cytoskeleton staining (f-actin) and intact nuclei (Hoechst). No increase in cell death, as measured with active caspase 3 (CC3), was observed after 7 days of culture inside the explant structures, only at the periphery of the explants, suggesting that NFC matrices are suitable for breast cancer explant *ex vivo* culture (Figures 1-6). Biotinylated human recombinant IL-2 in the ANFC-A matrix was not observed to have any adverse effects on epithelial cell viability, which confirms its safe use also for the further oncological studies (Figure 5, 6). The general proliferation level (Ki67) was, however, observed to be considerably low, but as the same was observed in the uncultured (day 0) samples and in Matrigel cultures, this may result more from the tumour type rather than from the culture conditions (Figures 7-8).

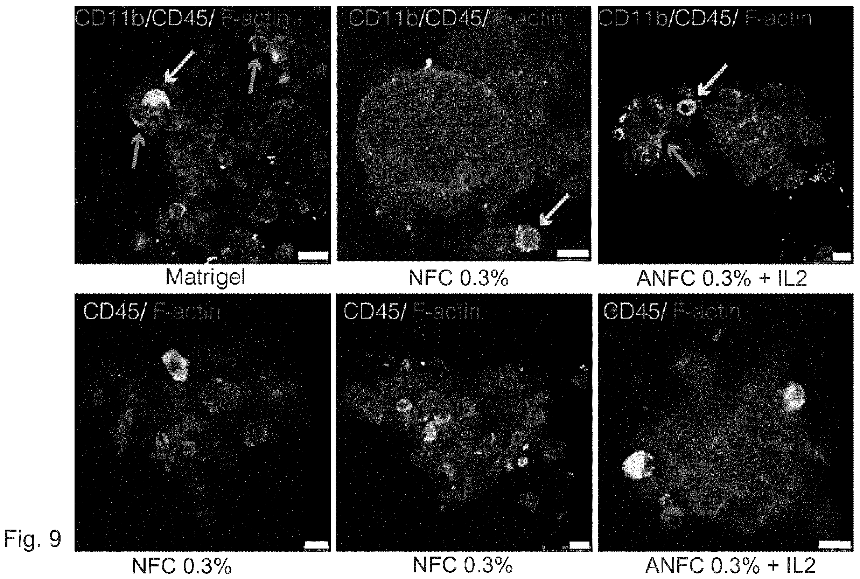

The immune cells were detected in every tested NFC matrix (Figure 9). However, the change in the matrix concentrations failed to prevent the loss of tumour and immune cells during the extensive washes of the immunofluorescent staining protocol. The samples were stained in the same 8-chamber slides, where the cells have been growing. The advantage of this is that it is possible to mount the cells in the same place where they have been growing, which is at the same time a microscope slide, so it is possible to avoid of moving the sample from one vessel to another. To be able to quantify immune cell survival and to monitor the effects of NFC matrices on different immune cell types, the next analyses were carried out with flow cytometry instead of IF-staining (UH). Due to the technical issues with the staining, it is difficult to state anything definitive about the exact quantities of the immune cells within the cultures.

### Effects of NFC on immune cells

### Flow analysis of PBMCs

The PDECs were replaced with naive immune cells, called blood peripheral mononuclear cells (PBMC), in order to monitor the direct effects of NFCs on immune cell numbers and immune cell types. The naive PBMC population is composed of roughly 60% T-cells, 15% B-cells, 5% NK-cells, and 20% monocytes, which can differentiate into macrophages, and dendritic cells in the tissues. Here, it was of interest to see whether all cell types are supported by NFC, are they activated, and whether monocytes differentiate into macrophages and dendritic cells. Cells were embedded into the tested NFCs and cultured for 5 days (5d).

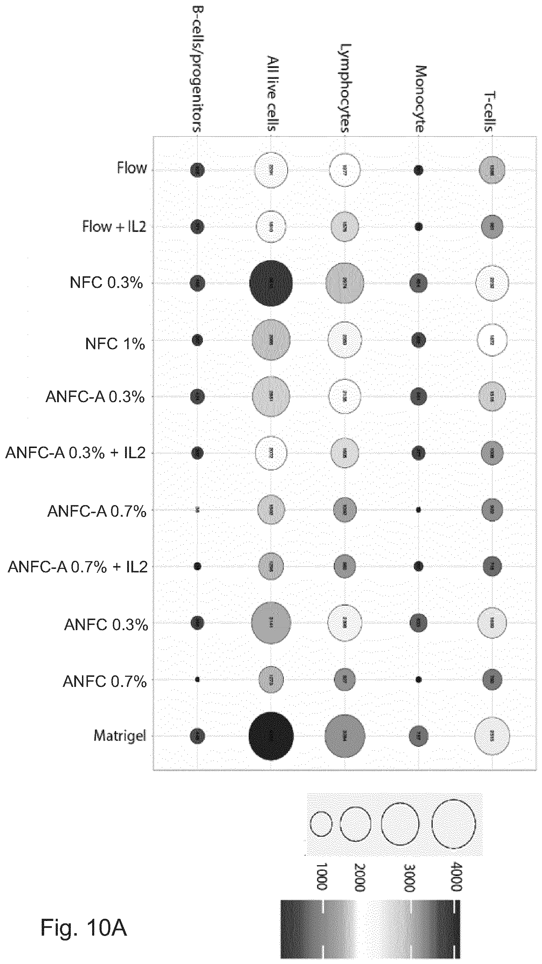

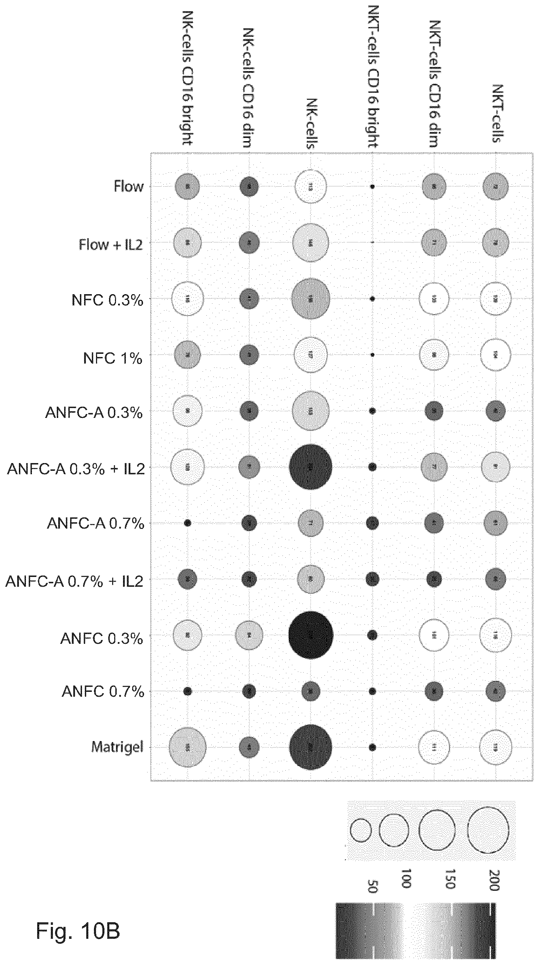

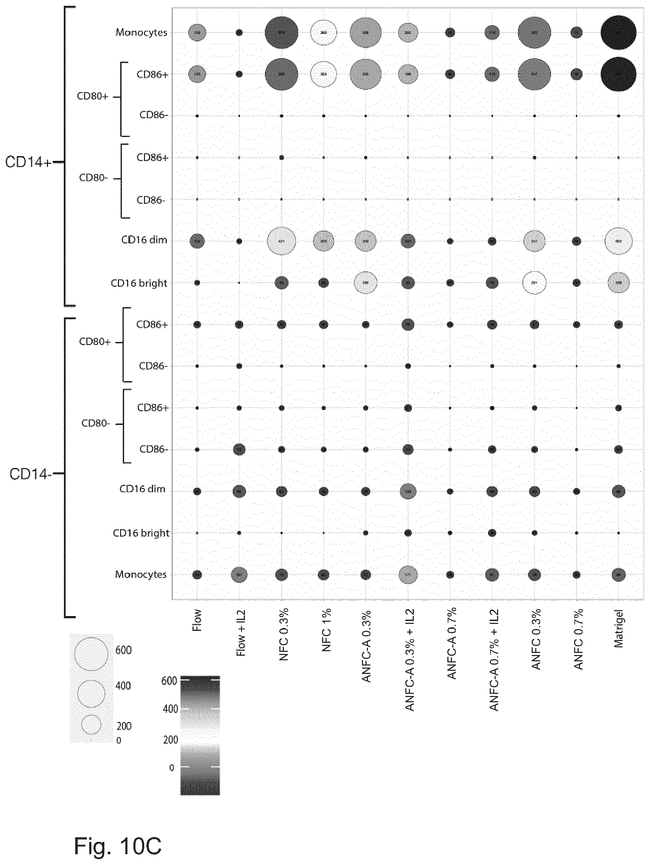

After the cultures, the NFCs were dissociated with GrowDase in +37C° for 1h and Matrigel samples were dissociated with Cultrex Organoid Harvesting solution in +4C ° for 1 h. Live cells were stained with flow cytometry antibody panels and analyzed with BD FACS Aria II. The experiment was repeated twice. In the first set of experiments the challenge was to dissociate the cells sufficiently from the matrices due to the imperfect performance of the GrowDase. The issues with the NFC degradation were seen in the results as lowered cell numbers compared to the Matrigel. Cultrex Organoid Harvesting solution dissociation had already been optimized for the present purposes earlier. For the 2^{nd} set of experiment the matrix degradation was optimized, and the concentration of the GrowDase enzyme was increased (see detailed protocol in 5.2 Protocols/ Flow Cytometry). Figure 10 shows the results from the 2^{nd} experiment as absolute cell numbers (Figure 10a-c). The size of the ball is relative to cell numbers as is the shade of grey. The bigger the size of the ball and darker the shade of grey is, the more immune cells of a particular type (the immune cell type is show in the Y-axis) is present in the sample (samples = matrix, which is indicated in the x-axis).
CD80+CD86+ Dendritic cell, Macrophage (M1)
CD80-CD86+ Monocyte, DC, Macrophage (M2)
CD80+CD86- Macrophage (M1)
CD80-CD86- Monocyte

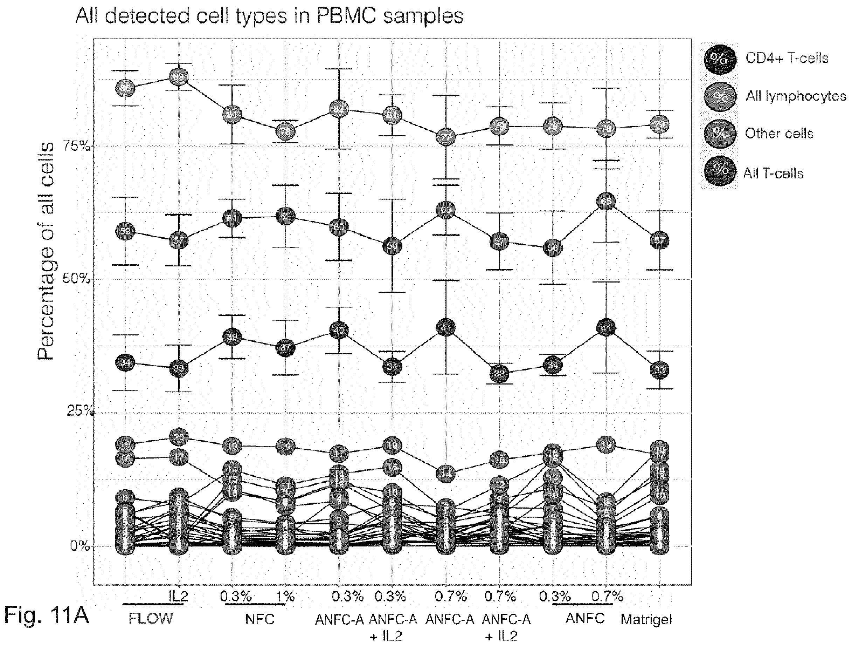

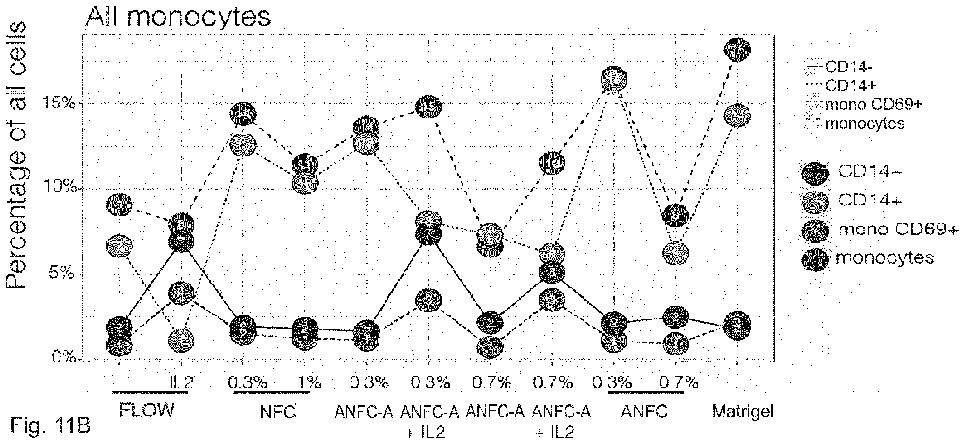

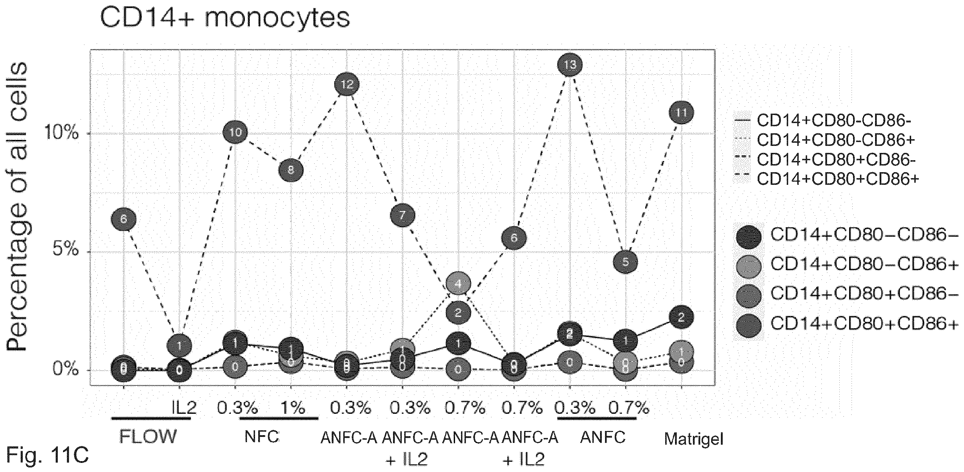

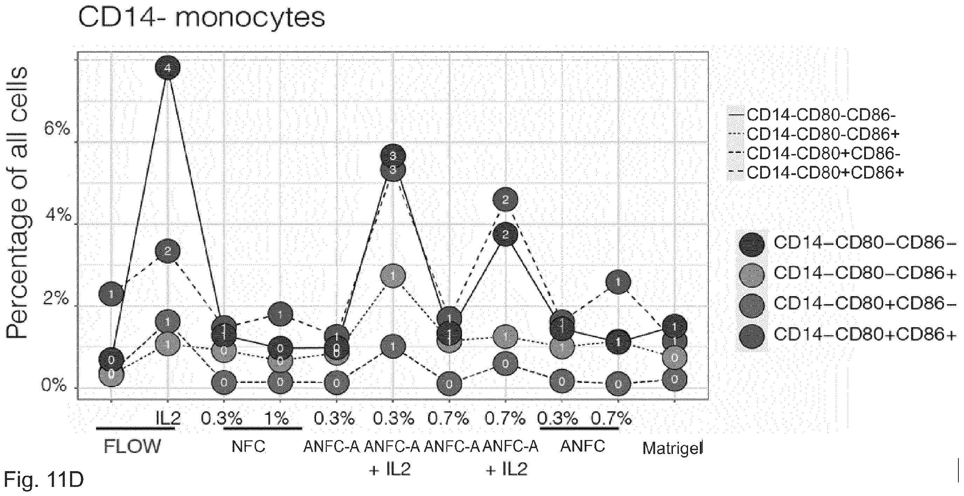

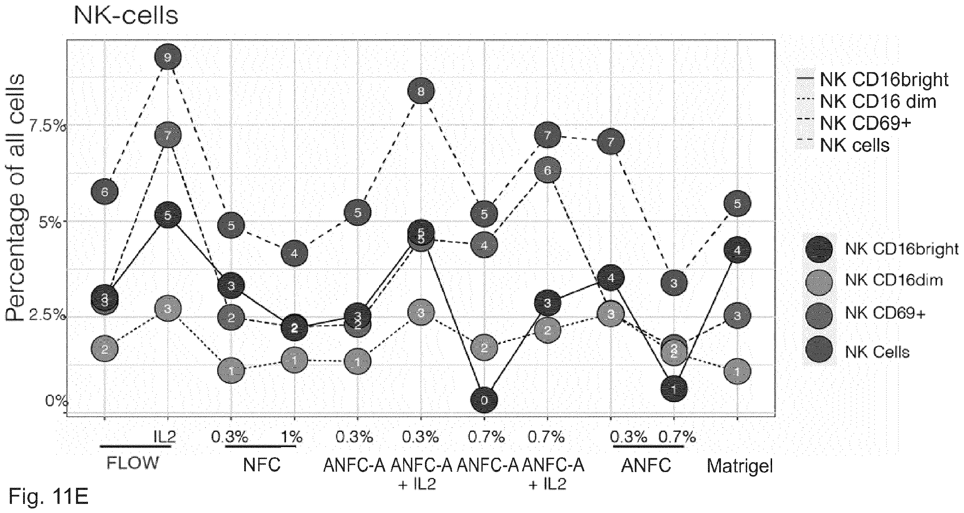

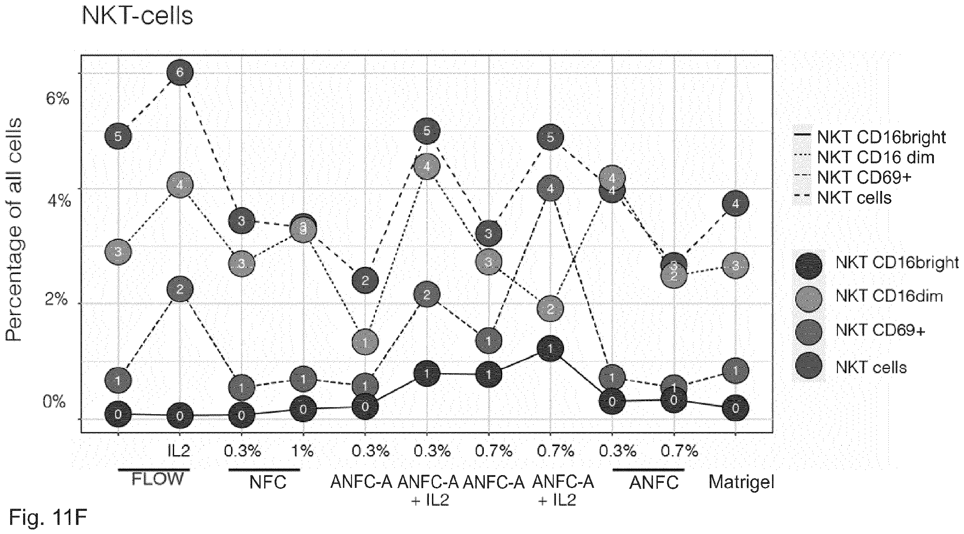

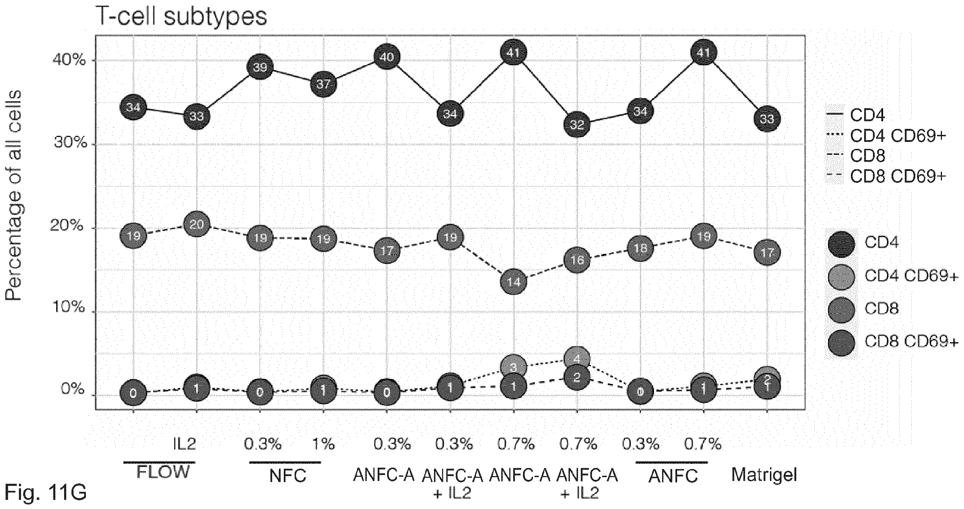

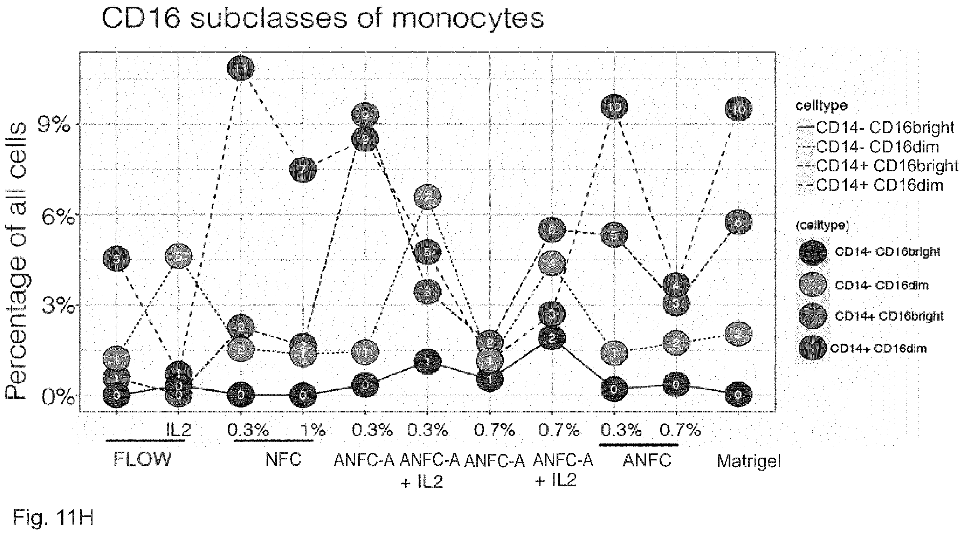

According to the FACS results each matrix had better performance in maintaining the PBMCs cells in ex vivo conditions compared to the traditional floating culture, which is supplemented with IL-2 (Figure 10). It is, however, hard to compare the performance of each matrix to each other due to the variation in matrix dissociation. Therefore, by normalizing each cell type to the total live cell number in each matrix, enabled to exclude the technical variation caused by the differences in matrix dissociation and to solely focus on the effect of each matrix on the immune cell types (Figure 11). The different immune cell types are shown with different shades of grey. The Y-axis shows the percentage of different immune cell types in each matrix (the % of cells are indicated in the ball). The different matrices are shown in the X-axis.

Different NFCs had a different ability to maintain the immune cell types in *ex vivo* conditions.

According to the FACS results the IL-2 functionalization had a positive effect on NK, NKT, and CD14- monocytes, which contains macrophages and dendritic cells.

On the contrary to the NK, NKT, and CD14- monocytes, the IL-2 supplementation appeared to have a negative effect on the CD14+ monocytes. This immune cell type was observed to be most prevalent in the low concentrated NFCs and the IL-2 functionalization lowered their abundance in 0.3% ANFC-A.

Of the nanocelluloses, T-cells were more prevalent in higher concentrated ANFC-A and ANFC gels.

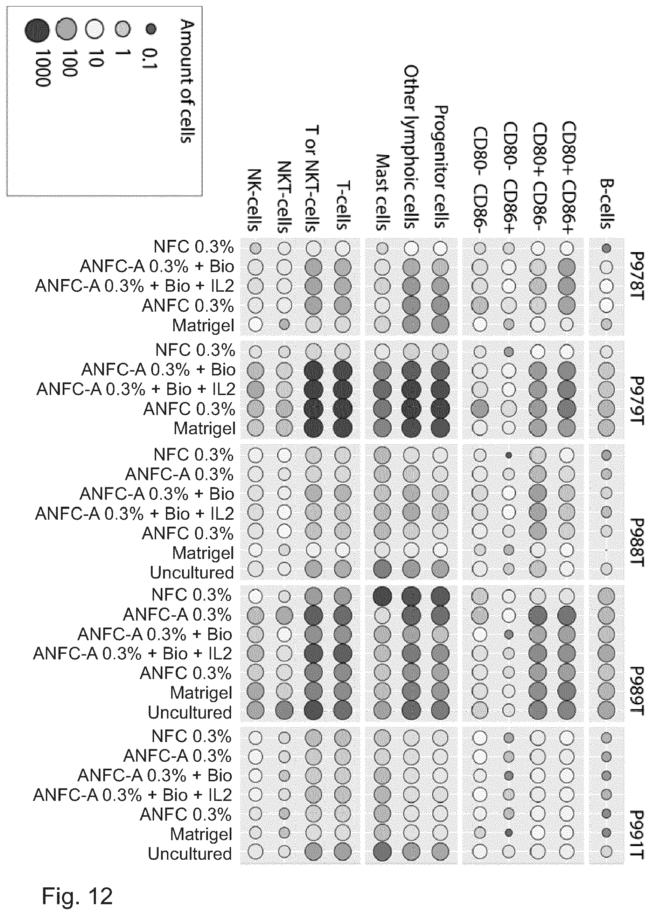

Tumours from five patients were analyzed with flow cytometry to estimate the number of live tumour resident immune cells and immune cell subtypes before and after 7 days of culture (Figure 12). Here the conditions were: Matrigel, NFC 0.3%, ANFC 0.3%, ANFC-A 0.3%, ANFC-A 0.3% with biotin, ANFC-A 0.3% with biotin and IL-2 functionalization. The amount of live immune cells was approximately 20% of the total amount of immune cells (CD45+) in fresh sample, and the ratio stayed the same after 7 days of culture. The presence of T-cells, B-cells, NK-cells and myeloid cells (population of monocytes, macrophages and dendritic cells) in 3D cultures after 7 days of culture could be confirmed (Figure 12). The patient derived immune cells were not preserved clearly better in any of the studied 0.3% matrices, but the result varied between patients as expected with patient heterogeneity. The Figure 12 illustrates the different immune cell types with the balls. The bigger the ball size and darker the shade of grey, more immune cells of that sort are present. More CD80 is expressed on macrophages and dendritic cells (DC). More CD86 is expressed on monocytes and DCs

Similarly, to the FACS results, the other measured cytokines (TNF-α, IL-6, IL-1β, and LAP [TGF-β1]) showed mainly patient specific variation in the immune cell composition. For example, higher IL-4 levels in two patients correlated with lower levels of TGFβ. The antagonistic effect between TGFβ and IL-4 is known from the literature. Additionally, two major trends were observed from the results. TNFα and IL-1β production was increased in the higher concentrated matrices, whereas the IL-6 and TGFβ production was observed to increase in the soft matrices overtime. These results are in line with the results from the FACS analysis, which showed that the different matrices affected differentially on the immune cell composition. The IL-6 and TGFβ are considered as suppressive cytokines and the production of these cytokines in the lower concentrated NFC as observed to correlate with the increase in the population of immunosuppressive monocyte. Higher concentrated NFCs supported more T-cells preservation (indicated by the FACS), which thus correlated with higher levels of proinflammatory cytokines observed in these materials.

The cytokine results are show with circular graphs. Different matrices are indicated with different shades of grey. The higher the level of a cytokine in a particular sample, the closer to the outer rim the shade reaches. The numbers indicate the amount of cytokine in pg/ml.

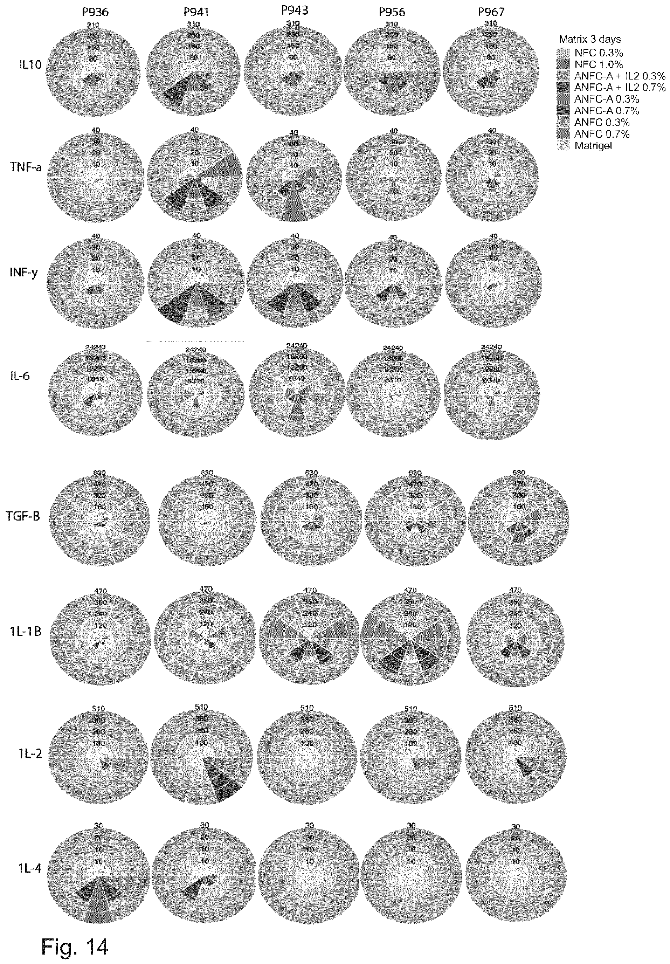

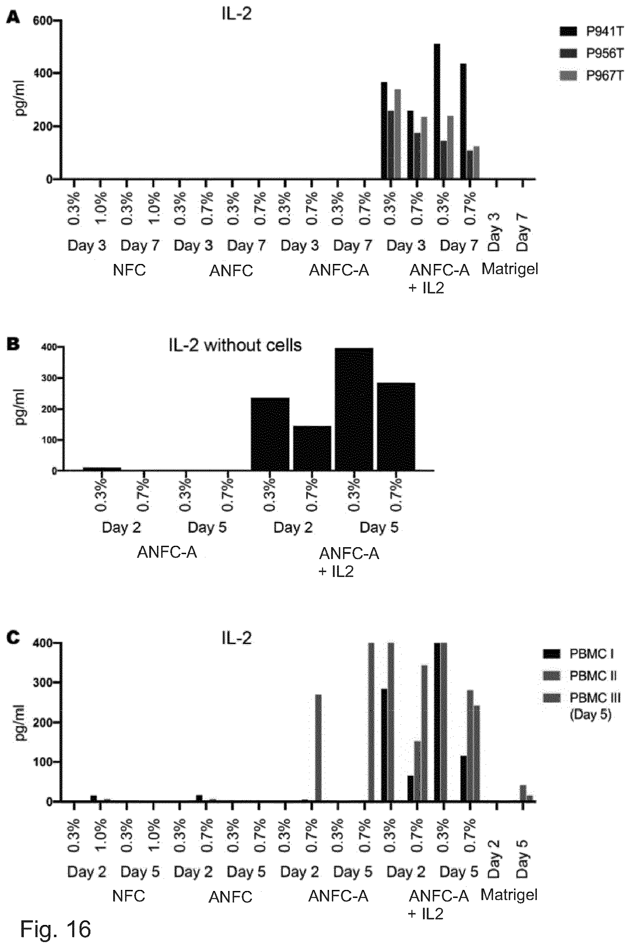

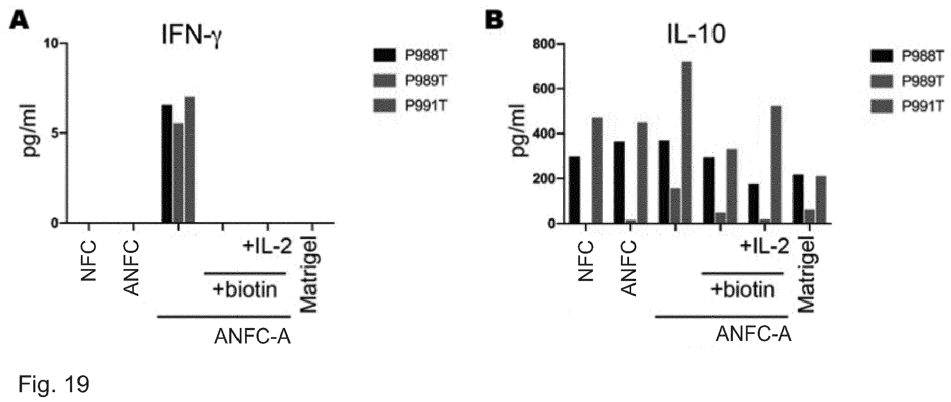

An inflammatory reaction caused by the ANFC-A matrix was seen with the PDEC cytokine profiling. The results revealed clear upregulation of IL-4, IFN-γ, and IL-10 in ANFC-A matrix (Figure 14). These cytokines are indicators of inflammatory response. IL-2 cytokine secretion cannot be measured from NFC-PDEC growth media due to the leakage of biotinylated IL-2 from the functionalized matrix (Figure 16). In order to prevent the unwanted inflammatory reaction, an additional biotin treatment step could be included after the matrix functionalization into the protocol in order to block the empty avidin sites and thus prevent these free avidin sites from stealing the biotin of the culture media. The addition of 1 µM biotin to PDEC growth media clearly decreased the amount of inflammatory cytokine IFN-γ in NFC cultures (Figure 19).

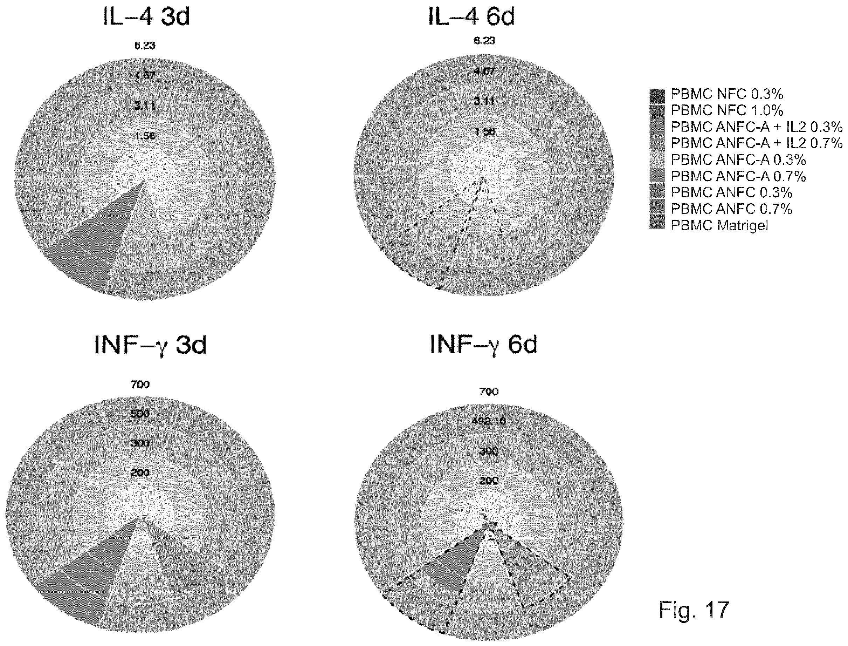

With PBMCs the inflammatory effect was mainly observed through increased IFN-γ cytokine levels (Figure 17). Additionally, IL-4 was observed to peak in the high concentrated ANFC-A without any additional biotin supplementation (Figure 17). This is consistent with the known literature, which proposes the inhibitory effect of biotin supplementation on IL-4 production in the PBMCS. The IL-2 functionalization gave a growth benefit to NK and NKT cells, which both produce IFN-γ, which might thus explain why IFN-γ is only seen in the functionalized matrices. Unlike with the PDECs only IL-10 production was seen to be dependent on the matrix stiffness in PBMCS. Also, no increase in any of the cytokine production was seen overtime, which may suggest an additional level of regulation between the immune cells and the tumour cells, which is absent from the PBMC cultures. The general decrease of cytokine production over time might result from media changes that might cause a gradual loss of cells over time, which are released from the matrix. Another reason might be, because some of these naive primary cells will commit to different lineages, as seen from the FACS results, and thus lose their regeneration capacity.

### Comprehensive analysis of the immuno-contexture in NFC-grown TIME-PDEC

### Analysis of the immune cell identity in NFC-grown TIME-PDECs

Single-cell RNA sequencing was used to resolve immune cell identity and to define the heterogeneity of human T-cells in PDEC cultures. The aim was to choose the most CD45+ preserving NFC condition and to sort the immune cells from two TIME-PDEC samples grown in NFC for one week and subject them to single cell sequencing analysis. NFC in 0.3% concentration was selected to be the most immune cell preserving condition based on the Figure 10a. First analysis was performed with following conditions: Uncultured (after collagenase treatment), and NFC 0.3% cultured. The number of conditions was kept as small as possible to ensure an adequate number of live immune cells for single-cell analysis. Minimum amount of live immune cells needed for the analysis is 10 000 cells per condition. In the first experiment, the amount of live immune cells in the uncultured sample was too low for single-cell sequencing. Since the overall CD45+ population in the uncultured sample was rather large, the tumour dissociation method was too harsh for the immune cells, since cells did not survive the treatment. For the next assay, gentler collagenase treatment for tumour dissociation method improved markedly the immune cell survival and the two samples were further processed for single-cell RNA sequencing.

### Detailed analysis of the tumour immune microenvironment in NFC grown TIME-PDECs

To see the more detailed effect of the microenvironment on the immune cells and simultaneously analyse the general impact of the *ex vivo* cultures on the gene expression profiles of the NFC grown PDECs, bulk RNA sequencing was performed. For the analysis, 5 patient samples were analysed. The chosen conditions were following: Uncultured (after collagenase treatment), Matrigel, NFC (1.0%/0.3%), ANFC (0.7%/0.3%) and ANFC-A (0.7%/0.3%) with and without IL-2 functionalization. Although the amount of sample was originally similar in each condition, the amount of RNA was very low in NFC-derived samples. Instead of RNEasy RNA extraction kit (Qiagen), Trizol extraction would be more suitable for NFC extractions yielding very high quantities of good quality RNA. This was only discovered after the project.

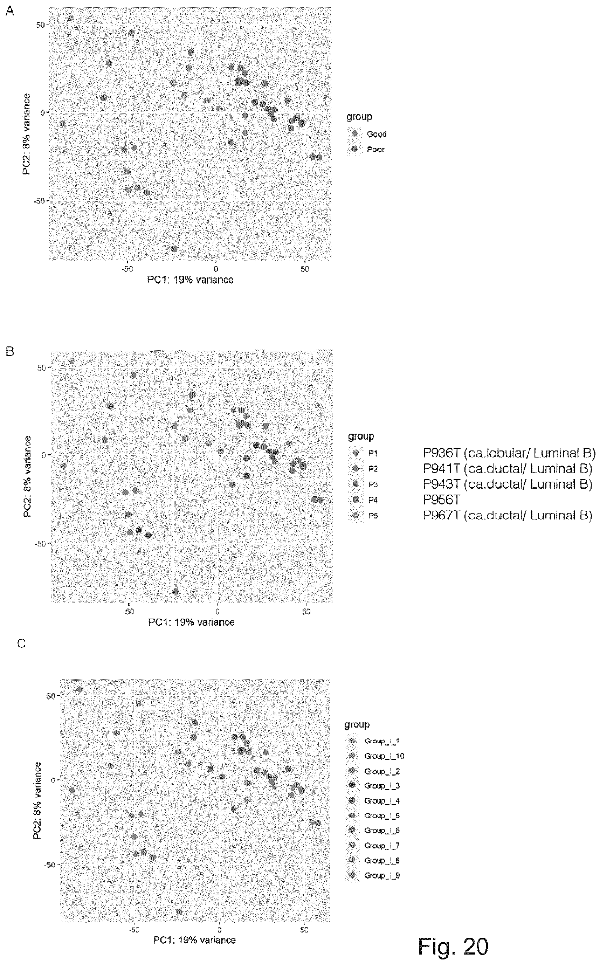

Some conditions did not have enough RNA for the analysis, which is why 5 replicates cannot be achieved from each condition. The results were analyzed using gene set enrichment analysis (UC San Diego & Broad Institute) and the Molecular Signature Database and visualized using Cytoscape (v.3.7.2) and the enrichment map plug-in. Here, the changes in gene expression were analysed by grouping the genes into gene sets (Hallmark [50 gene sets], PID [196 gene sets], Biocarta [288 gene sets], and collection of immune cell function related gene sets) and evaluating the enrichment of these gene sets between different conditions. The analysis showed which gene sets were enriched or decreased after culturing the PDECs in NFCs for 7 days. Patient material is very heterogeneous, and it was difficult to obtain enough RNA from every culture condition of each patient. 3 biological replicates/conditions were thus kept as the minimum for the sequencing. Due to the variability in the RNA quality, the data from the sequencing was first visualized with principal component analysis (PCA) to see if the RNA quality affected the sample clustering (Fig. 20). When the RNA integrity values were high (RIN >7 = good quality RNA), the samples distributed more in the 2D space as represented by red dots compared to the blue dots, which represent the samples with poor RNA quality (RIN <7). The difference in the distribution suggests that from the poor-quality samples, less reads were obtained from the highly expressed genes compared to the good quality samples and the diversity of the libraries were thus lower in these samples (Fig 20 A). The difference in the RNA quality was considering in the data analysis. In the 2^{nd} PCA graph the samples seemed to cluster also slightly according to the patient, which is often seen with the tumour samples (Fig 20 B). When investigating the effect from the culture conditions (matrix) on the transcriptomics (3^{rd} PCA in the Fig. 20), both the quality differences and the sample origin was taken into consideration.

The investigated gene sets included profiles from major signalling pathways and multiple immune cell profiles. To see the general changes in the transcriptome between 3D-cultured PDECS and uncultured samples, all the 578 gene sets between these two groups were first compared. Each ball represents a single gene set and each sector inside a ball represents an individual growth condition (matrix). Gene sets, which were significantly (qFDR = <0.2) upregulated in the 3D cultures were shown in red, whereas the significantly downregulated gene sets were indicated in blue. Less than 150 gene sets from the total of 535 sets were significantly enriched (FDR q < 0.2) in the 3D matrices compared to the uncultured samples. Next, each matrix was separately compared to the uncultured samples to see the matrix specific gene expression profiles. If the matrix has a strong effect on the transcriptional profiles of the sample, more gene sets are expected to be enriched when compared to the uncultured sample. This was seen, for example, from Matrigel, which is known to contain a lot of growth factors, which have an impact on the transcriptional profiles of the sample. The effect of Matrigel on the gene expression profiles was seen also from the PCA diagram, where Matrigel samples were seen to cluster separately from the NFC samples which confirms that the matrix clearly affects the gene expression profiles of the samples (Fig 20 B). Similarly, the 0.3% NFC matrix was observed to cluster separately from the rest of the NFCs in most of the samples. The upregulation on multiple signalling pathways were observed in the NFCs and Matrigel in general compared to the original tumour. The upregulated gene signatures included e.g. following pathways: TGFβ, mTOR, KRAS, WNT. The only matrix, which failed to upregulate these pathways was 1% NFC.

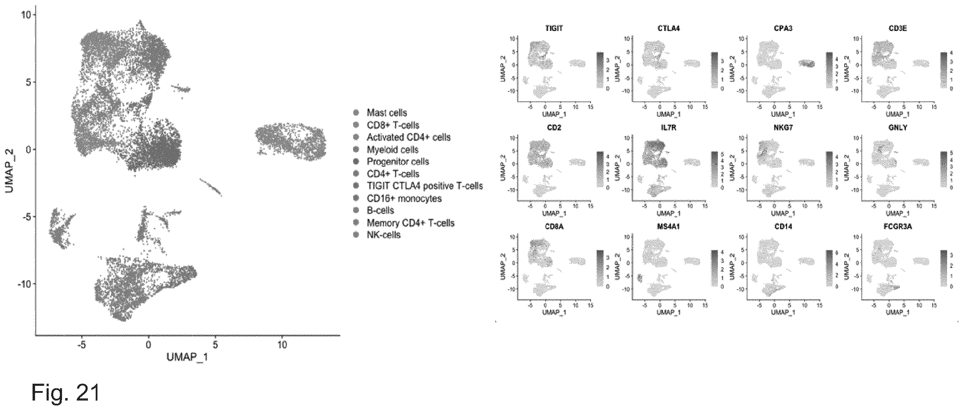

The most detailed immune cell analysis with scRNASEQ was done for two patient samples P981T and P982T. The immune cells from the fresh uncultured tumours were isolated the based on their CD45 expression with fluorescence-activated cell sorting (FACS) The isolated immune cells were subjected to sequencing. The rest of the tumour was embedded in 0.3% NFC and cultured in *ex vivo* conditions for a week. The immune cells were isolated from the cultured sample based on their CD45 expression. The isolated immune cells were subjected to sequencing. The results from the scRNASEQ shows that all the immune cell types were represented in the cultures after one week of cultures. The different immune cell subtypes were identified based on their expression profiles (Fig. 21). The cell numbers vary between cultured and uncultured samples, which affects the coverage of the samples. This will affect to the results by showing lower expressed genes better in a 3D cultured samples. As the overall cell number is lower in 3D cultured compared to the uncultured samples, the sequencing coverage will be divided with smaller number of cells, which will result in deeper sequencing and thus show better also the low expressed genes in 3D samples. In the uncultured samples, where the sequencing capacity is divided with higher number of cells the depth of sequencing will thus be lower, because the sequencing capacity is divided between higher number of cells. The difference in the coverage will be, however, taken into consideration with FIMM inbuild pipeline for 10x Genomics, which makes the data comparable between different samples despite of the coverage difference.

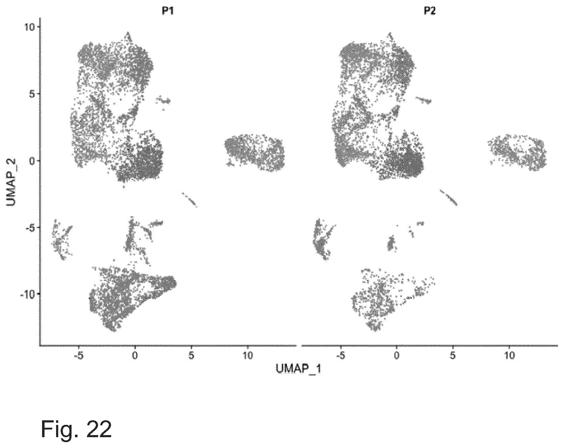

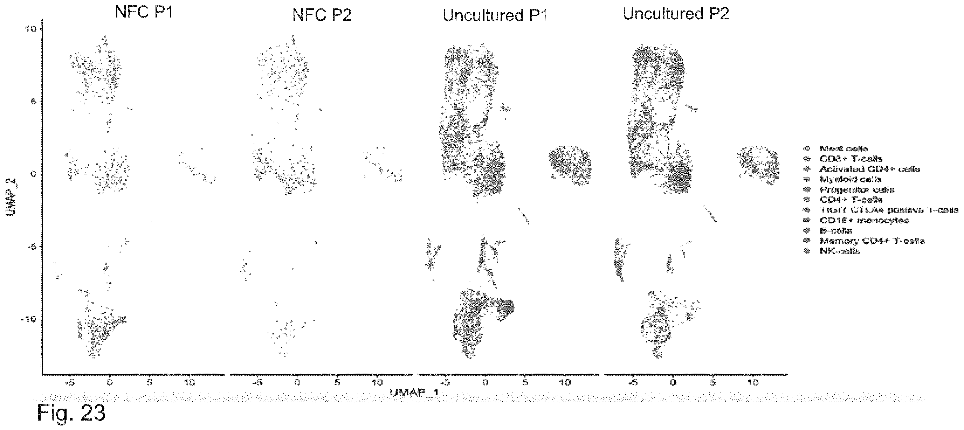

The scRNASEQ data revealed clear differences in the immune cell contexture between the two patients (Fig. 22, 23). Both samples were luminal B type of tumours, but the patient P981T (P1) was a ductal and the P982T (P2) a lobular carcinoma.

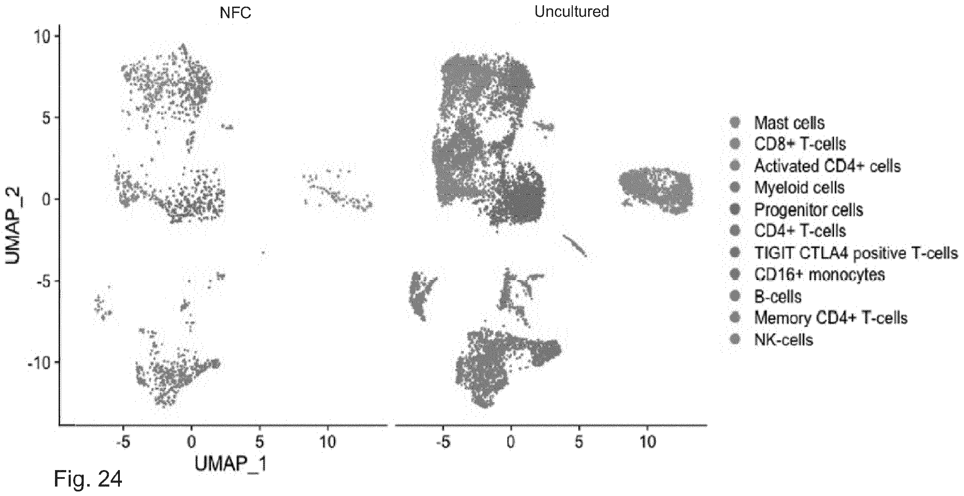

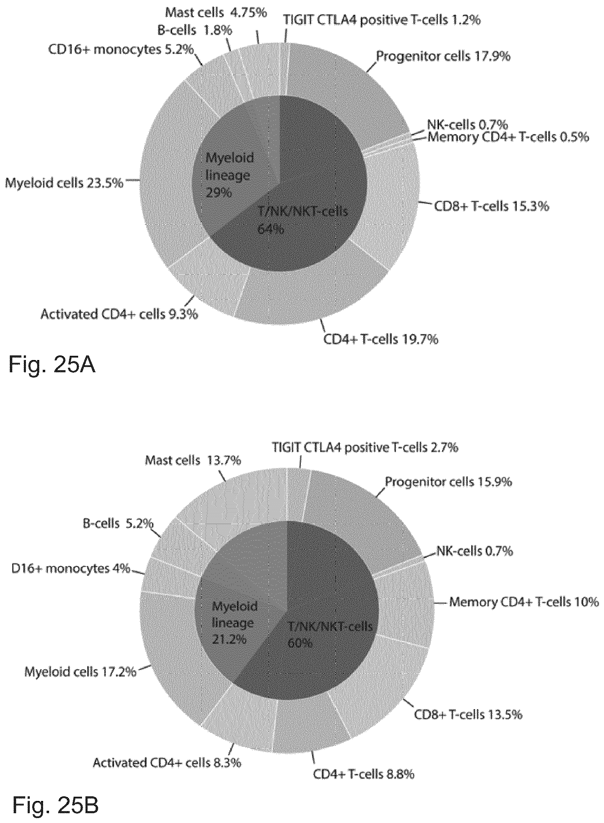

The comparison between uncultured and 3D cultured samples showed slight changes in the immune cell composition (Fig. 24). Detailed immune cell comparisons revealed that the most obvious change was observed in the disappearance of a cluster that mostly expressed genes linked to some T-cells subtypes from 3D cultures. Also, the B-cell and mast cell populations were decreased in the 3D cultures, whereas a slight increase in the myeloid cell populations were observed in the 3D cultures compared to uncultured samples. The results are consistent with the FACS results by showing that the low NFC concentration is more beneficial for the myeloid cells compared to the T-cells, which were more numerous in higher polymer NFC concentrations. Altogether the results suggest that the low polymer NFC concentrations recapitulates conditions that supports tumour cell mediated immune cell evasion shown by the changes in the cytokine production and immune cell composition. Figure 25 presents proportions of the immune cell types in the uncultured (25B) and 3D cultured (25A) samples. Two patient samples are pooled together

### Materials and methods

### PDEC cultures

Primary breast cancer tissue was processed into small explants (previously described in Haikala et al. (Pharmacological reactivation of MYC-dependent apoptosis induces susceptibility to anti-PD-1 immunotherapy, Nature Communications, 2019, 10:620; https://www.nature.com/articles/s41467-019-08541-2) and embedded in 3D matrices. In total 40 µl of matrix, forming a dome, per well was cultured in 500 µl of MammoCult growth media (STEMCELL, #05620) in ex vivo for 7 days on an 8-well chamber slides (Thermo Fisher Scientific, 177402). The tested 3D matrices were following: NFC (1.0%/0.3%), ANFC (0.7%/0.3%) and ANFC-A (0.7%/0.3%), and as a control undiluted Matrigel. The Matrigel was used as 40 µl of matrix together with 500 µl of MammoCult growth media in a well. The NFCs were diluted to the desired concentrations according to the manufacturer's instructions. ANFC-A was functionalized with human recombinant biotinylated IL-2 (2 ng/ml) (BT202-025/CF, R&D Systems). 50 µl of IL-2 diluted in 1x PBS was mixed with 300 µl (0.3%) or 700 µl (0.7%) of NFC and incubated for 1 hour at RT. Functionalized matrix was mixed with culture media to adjust the correct concentrations (550 µl for 0.3% and 150 µl for 0.7%). Finally, the explants were mixed with the matrix similarly to other studied matrices. Three biological replicates of patient derived explant cultures (PDEC) were processed in each condition.

### PBMC cultures

Buffy coat from one donor was obtained from FRC blood service, Helsinki. Human peripheral blood mononuclear cells were isolated with Ficol-Paque density centrifugation and stored at -145°C. Cells were quickly thawed and mixed with diluted NFC so that one 8-well chamber slides well contained 2 million cells in 40 µl of matrix. 500 µl of media was added on top, and when media was replenished at the day 3, sample was collected for cytokine profiling. At day 5 the growth media was collected for cytokine profiling, and 3D culture was further processed for flow cytometry analysis. In addition to 3D culture, 2 million PBMCs were cultured as a floating culture, where cells were added on a low adhesion 6-well plate in 2 ml of growth media, with or without IL-2 supplementation (2 ng/ml). In floating culture, media was not changed during 5 days of culture.

### Immunofluorescent staining

The effects of the 3D culturing on explant viability, proliferation and apoptosis, was analyzed with immunofluorescent staining. The cells were stained with the immunofluorescence markers ki67 (ab15588) and CC3 (Cell signalling # 9661) accordingly. First, cells were fixed with 4 % PFA for 10 min, washed 3x10 min with immunofluorescence (IF) buffer (7.7 mM NaN₃, 0.1% BSA, 0.2% Triton-X, 0.05% Tween20), and then permeabilized with 0.25% Triton-X in PBS for 20 min and washed again. Nonspecific binding sites were blocked by incubating the cells for 1h at RT with 10% normal goat serum (cat. no 16210064, Thermo Fisher Scientific) in IF buffer. Primary antibody was then diluted in blocking buffer and incubated statically with the cells for 24h at +4 °C. After incubation with the primary antibody, the cells were washed 3x20 min with IF buffer in gentle rocking at RT. Secondary antibody against the primary antibody and F-actin was diluted in blocking buffer and incubated with the cells 15-17 h at +4°C protected from light. F-actin (cell cytoskeleton) was labelled with AlexaFluor Phalloidin 546 (Invitrogen, A22283) and cell nuclei with Hoechst. Hoechst staining was 10 minutes. After the 2^{nd} antibody incubation the samples were washed twice with IF-buffer and mounted with Immu-Mount.

### Confocal microscopy

The immunostained cell structures were imaged with a confocal laser scanning microscope (Leica TCS SP8 MP CARS with a HC PL APO 40x/1.10 water CS2 objective, Leica Microsystems). The pinhole was set to 1.00 AU and 405 nm diode, 488 nm argon and 561 nm DPSS lasers were used.

### Cytokine profiling

Media (1 ml) collected from NFC-grown PDECs or PBMCs at day 3 and day 7 timepoints was stored at -80°C until the day of analysis. The volume of collected growth medium does not matter, because the same volume (100 µl) from each sample was used in the analysis. Cell and matrix debris were removed after thawing by centrifugation (10 min, 10 000G, RT). Cytokines secreted to growth media were analyzed with R&D DuoSetELISA kits (IL2: DY202-05, IL-10: DY217B-05, TNF-α: DY210-05, IFN-γ- DY285B-05, IL-4: DY204-05, IL-6: DY206-05, IL-1β/IL1F2: DY201-05, LAP (TGF-β1): DY246-05) together with DuoSet Ancillary Reagent Kit 2 (DY008), according to manufacturer's protocol. Briefly, plates were coated with capture antibody overnight at RT, washed 3 times and blocked with 1% BSA in PBS for 1 hour. Samples diluted in growth media were added and incubated for 2 hours at RT. After 3 washes, detection antibody was added and incubated for 2 hours at RT. After washes, Streptavidin-HRP was added and incubated for 20 min at RT, protected from light. After washes, Substrate solution was added for 20 min, protected from light, after which the reaction was stopped with stop solution and absorbance was immediately determined using a microplate reader set to 450 nm, and 540 nm to subtract background (Omega Fluostar, BMG Labtech).

### Flow cytometry

NFC-grown PBMCs were stained for immunophenotyping by flow cytometry with lymphocyte and macrophage panel antibodies. First, 40 µl of NFC-PBMC were dissociated by adding 250 µl of diluted GrowDase enzyme per well and incubated at +37°C for 1 hour.

A 1:2 dilution of the GrowDase enzyme [stock = 10 mg/ml] was made resulting a working concentration of 5 mg/ml. From this dilution 250 µl was used to degrade the 1% and 0.7% gels.

For the 0.3% gels an enzyme dilution of 1:3 was made, which forms a working concentration of 3.3 mg/ml. From thus dilution 250 µl was used to degrade the 0.3% gels.

This amount of enzyme could dissociate the NFC efficiently in 1 hour, without harming the cells. Matrigel Growth Factor Reduced Basement Membrane Matrix (Corning, 354230) was dissociated with 400 µl/well of undiluted Cultrex Organoid Harvesting Solution (Nordic Biosite, 3700-100-01) at +4°C for 1 h. Floating culture cells were collected by pipetting and growth media was removed by centrifugation. Harvested cells were divided into two sets, which were immunolabeled with either Panel 1 or Panel 2 antibody mixture (1 million cells per condition) for 1.5 hours (Table 1). SYTOX green dead cell marker (Thermo Fisher Scientific, S34860) and Countbright Absolute counting beads (Invitrogen, C36950) were added to sample tubes and the cell counts were normalized to beads counted during the sample acquisition performed with BD FACS Aria II (BD Biosciences). UltraComp eBeads Plus Compensation Beads (Invitrogen, 01-3333-42) were labeled with single antibody-conjugates for signal compensation settings. The data were analyzed with FlowJo version 10.7.

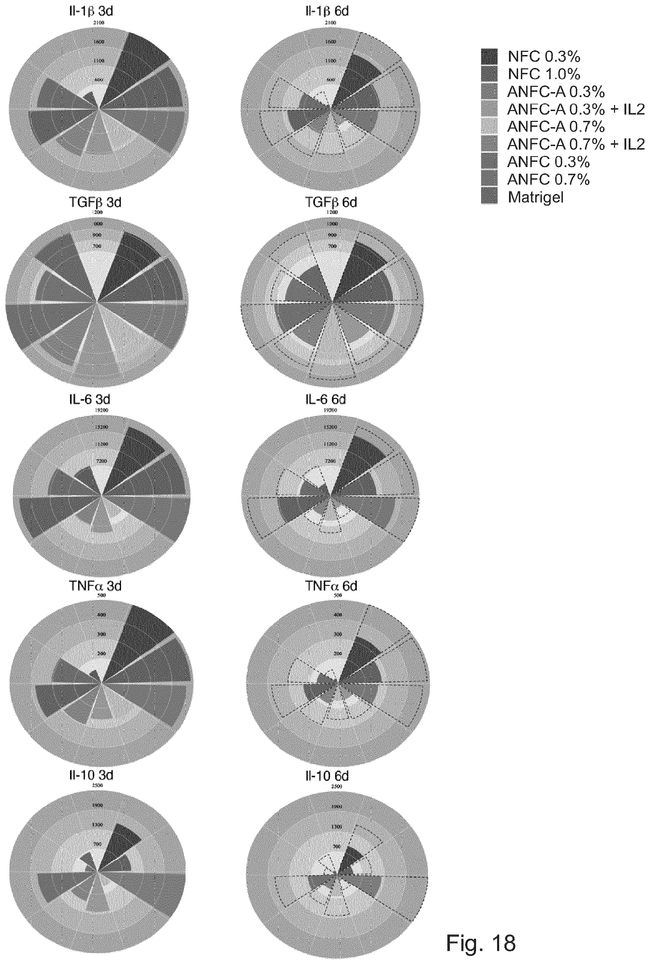

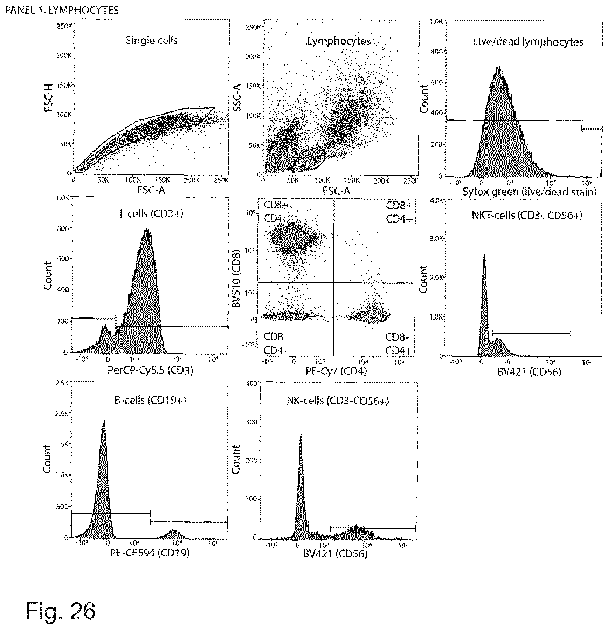

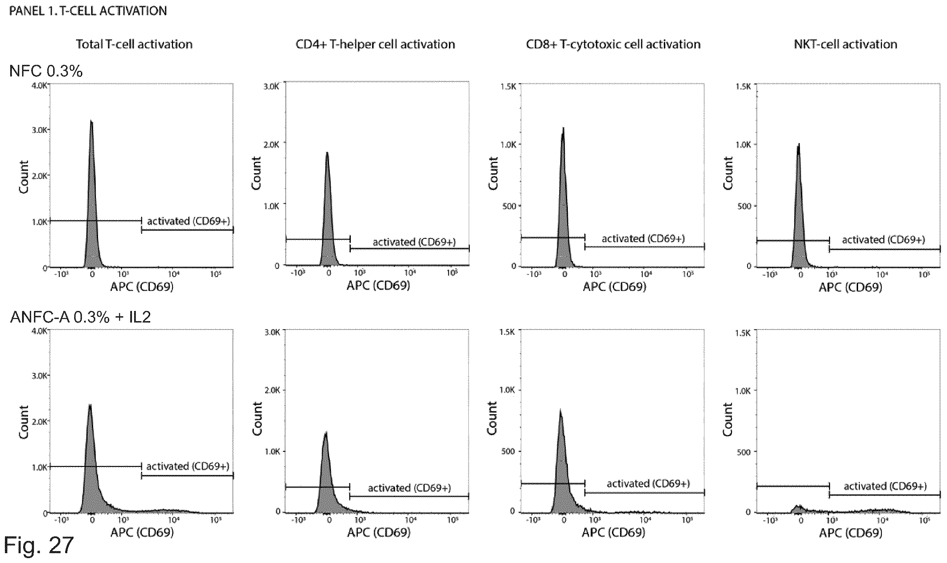

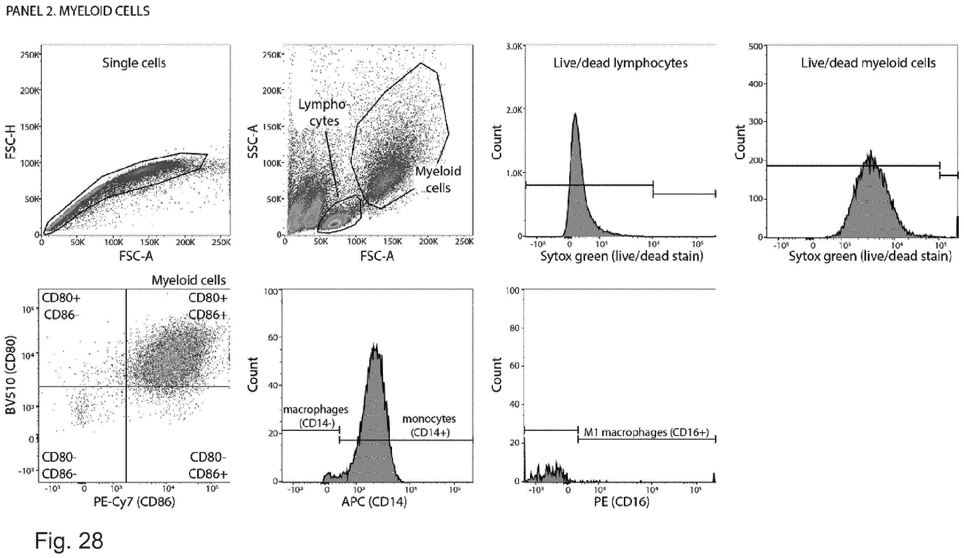

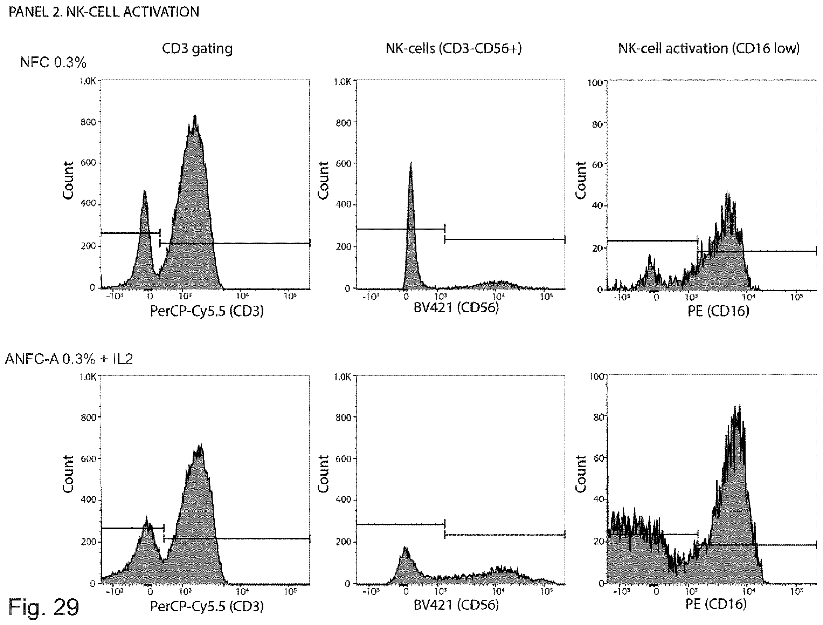

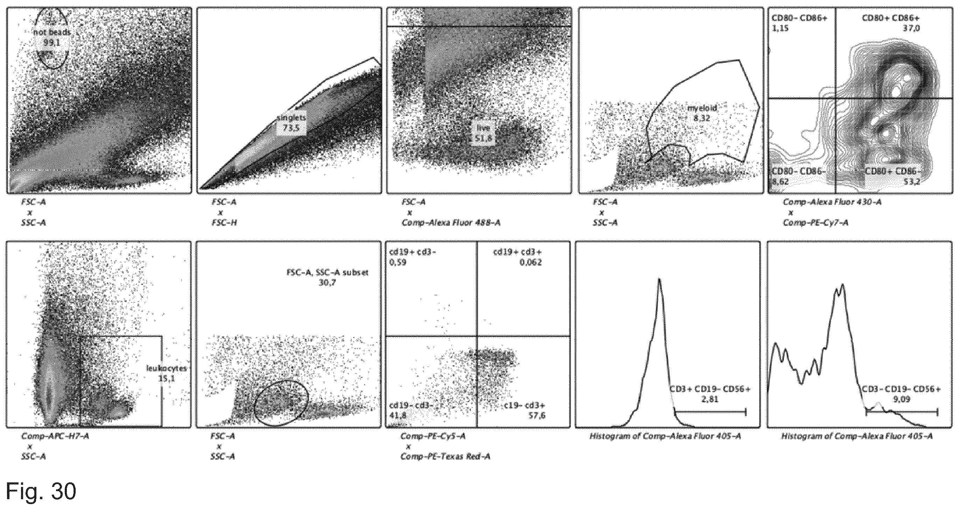

The following gating strategies were used for the identification of PBMC lymphocyte and monocyte populations (see Figures 18-21). Lymphocyte and myeloid cell populations were identified based on size on forward scatter vs. side scatter dot blot. Dead cells were excluded based on SYTOX green stain. T-cells were identified as CD3 positive, and from these the subtypes helper-T-cells as CD4 positive, cytotoxic T-cells as CD8 positive, and natural killer T-cells as CD56 positive. NK-cells were identified as CD3-CD56+ lymphocyte population. Activated NK-cells start to lose CD16 marker and activated T-cells start to express CD69 marker. B-cells were identified as CD19 positive population. Myeloid cells (monocytes, macrophages and dendritic cells) express high CD80 and high CD86. From these the monocyte population was identified with CD14 marker. CD14 negative cells were macrophages and dendritic cells, and from these the CD16 expressing cells were identified to be M1 class macrophages. The gating strategy for both PBMCs are shown in Figures 26-30.

**Table 1.**

| **Panel 1 (Lymphocytes)** | | |
|---|---|---|
| *Antibody-conjugate* | *microliters*/ *million cells* | *Clone* & *Manufacturer* |
| CD3-PerCP-Cy5.5 | 5 | Clone SK7; BD Biosciences 332771 |
| CD4-PE-Cy7 | 3 | Clone RPA-T4, BD Biosciences 560649 |
| CD8-BV510 | 3 | Clone SK1, BD Biosciences 563919 |
| CD19-PE-CF594 | 1 | Clone HIB19 (RUO), BD Biosciences 562321 |
| CD69-APC | 5 | Clone L78 (ASR), BD Biosciences 654663 |
| CD56-BV421 | 1 | Clone NCAM16.2, BD Biosciences 562751 |
| Sytox Green | 1 | Thermo Fisher Scientific S34860 |

| **Panel 2 (Monocytes)** | | |
|---|---|---|
| *Antibody-conjugate* | *microliters*/ *million cells* | *Clone* & *Manufacturer* |
| CD3-PerCP-Cy5.5 | 5 | Clone SK7; BD Biosciences 332771 |
| CD56-BV421 | 1 | Clone NCAM16.2, BD Biosciences 562751 |
| CD14-APC | 1 | Clone M5E2 (RUO), BD Biosciences 555399 |
| CD16-PE | 1 | Clone B73.1, BD Biosciences 561313 |
| CD80-BV510 | 1 | Clone L307.4, BD Biosciences 563084 |
| CD86-PE-Cy7 | 1 | Clone 2331, BD Biosciences 561128 |
| Sytox Green | 1 | Thermo Fisher Scientific S34860 |

PDECs from 3 patients were analyzed with flow cytometry to have an estimation of immune cell contexture and viability in the samples after 7 days of growth. Following growth conditions were used: Matrigel, NFC 0.3%, ANFC 0.3%, ANFC-A, and ANFC-A 0.3% with biotin and with or without IL-2 functionalization. 1 µM biotin (Sigma, B4639-1G) was added to growth media in ANFC-A samples. Matrices were dissociated as described above. Harvested cells were immunolabeled with Panel 3 antibody mixture (table 2) for 45 minutes. SYTOX green dead cell marker (Thermo Fisher Scientific, S34860) and Countbright Absolute counting beads (Invitrogen, C36950) were added to sample tubes and the cell counts were normalized to beads counted during the sample acquisition performed with BD FACS Aria II (BD Biosciences). UltraComp eBeads Plus Compensation Beads (Invitrogen, 01-3333-42) were labeled with single antibody-conjugates for signal compensation settings. The data were analyzed with FlowJo version 10.7.

**Table 2**

| **Panel 3 (Immune cells in PDECs)** | | |
|---|---|---|
| *Antibody-conjugate* | *microliters*/ *million cells* | *Clone* & *Manufacturer* |
| CD45-APC-H7 | 3 | |
| CD3-PerCP-Cy5.5 | 5 | Clone SK7; BD Biosciences 332771 |
| CD56-BV421 | 2 | Clone NCAM16.2, BD Biosciences 562751 |
| CD19-PE-CF594 | 2 | Clone HIB19 (RUO), BD Biosciences 562321 |
| CD80-BV510 | 1 | Clone L307.4, BD Biosciences 563084 |
| CD86-PE-Cy7 | 1 | Clone 2331, BD Biosciences 561128 |
| Sytox Green | 1 | Thermo Fisher Scientific S34860 |

The following gating strategies were used for the identification of PDEC lymphocyte and monocyte populations (see Figure 23). First, debris and counting beads were removed based on size on FCS vs SSC blot, and singlets were separated from FSC-H vs FCS-A blot. Dead cells were excluded based on SYTOX green stain. CD45 positive immune cells were separated from CD45 negative cancer cells. T-cells were identified as CD3 positive, and natural killer T-cells as CD3 and CD56 positive cells. NK-cells were identified as CD3-CD56+ lymphocyte population. From CD3 negative cells, B-cells were identified as CD19 positive population and myeloid cells (monocytes, macrophages and dendritic cells) as high CD80 and high CD86 expressing cells. To determine live/dead immune cells, cells were additionally first selected based on CD45 expression and then separated to SYTOX green positive (dead) and negative (live) populations (not shown).

### Bulk RNA sequencing and data-analysis

RNA from 5 patient samples was isolated after 7 days of culture with RNeasy Mini kit (Qiagen 74106) and DNA was removed with Zymo RNA clean & concentrator kit (R1019). As a control, part of the tumour was frozen without culturing, after collagenase treatment (uncultured). The selected conditions were Matrigel, NFC (1.0%/0.3%), ANFC (0.7%/0.3%) and ANFC-A (0.7%/0.3%) with and without IL-2 functionalization. Before extraction, media was collected for cytokine analysis and the rest of the sample, without matrix degradation, was frozen at -80°C until further processing. Thawed samples were lysed with 350 µl of buffer RLT with betamercaptoethanol and homogenized by passing the samples through 20G needle. 350 µl of 70% ethanol was added and the samples were transferred to spin columns. After washes, samples were eluted with 30 µl of RNAse-free water. DNA digestion and RNA purification and concentration were done immediately after extraction with a Rna Clean & Concentrator^{™} kit (Zymo Research, BioSite-R1013). RNA was eluted with RNAse-free water and the concentration was measured with Nanodrop. RNA was frozen at -80°C until analysis. RNA concentration confirmation with Qubit, RNA quality check with TapeStation, and bulk RNA sequencing with Illumina NextSeq 500 were done as a service provided by the Biomedicum Functional Genomics Unit at the Helsinki Institute of Life Science and Biocenter Finland at the University of Helsinki. Samples with no detectable RNA levels were excluded from the analysis. The data from the Bulk RNA sequencing will be analyzed using Gene Set Enrichment Analysis (UC San Diego & Broad Institute) and Molecular Signature Database.

### Single-cell RNA sequencing

Fresh tumour samples from 2 patients were digested overnight with collagenase at +37°C with gentle shaking. The following day, the day 0 sample (uncultured) was processed for flow cytometry analysis and the rest of the sample was cultured in NFC 0.3% for 7 days, as described above. The cells were dissociated using a filter cap tube. Harvested cells were immunolabeled with CD45 antibody (3 µl/sample) for 45 min and cell sorting was done with BD Influx Cell sorter, as a service from Biomedicum Flow Cytometry Unit (HiLIFE, University of Helsinki). Cells were collected in PBS (without Mg₂/Ca₂)⁺ 0.04% BSA. Single-cell RNA sequencing was done as a service at FIMM Single-Cell Analytics unit.

Single cell sequencing and analysis was performed together with Finland Institute of Molecular Medicine using 10x protocols and software.

Gene-barcode-matrixes of all four samples were aggregated with 10x protocol. Aggregated gene-barcode-matrixes were used for further analysis.

Individual cells were then filtered with following criteria for obtaining healthy and reliable cells without doublets. Percentage of mitochondrial genes was set under 10%, number of individual genes above 200 and under 5000, UMI above 500 and under 30 000. After removal unhealthy cells and doublets, total of 12 188 cells were selected for further analysis. There were 932 cells from donor 1 in NFC 0.3% and 5581 cells in the uncultured sample from the same donor. In NFC 0.3% from the second donor 541 cells were left while the uncultured sample from the same patient provided us with 5134 healthy cells. Differences in cell numbers between uncultured and NFC sample were taken into account in the main analysis and also in the graphical representation. Ten times difference in the cell numbers creates a bias in the sequencing depths between samples. Sequencing depth is a parameter that describes how in detail each cell is analysed. If the depth is great, even the minor mRNA amounts are sequenced from a cell. If the depth is more shallow, only most commonly expressed mRNA are detected. The depth is the limit how in detailed the immune cell subtypes can be detected. The sequencing run has a limited capacity to recognize n mrna reads per sample where n is constant between samples. This may be described in very simple mathematical formula: n = cells x sequencing depth. Thus, if there are more cells, a smaller amount of information per cell is obtained. If there are very few cells, the amount of information per cell is greater and subtypes are detected in greater detail. This bias has been taken into account with preanalysis. The sequencing depth is downscaled in all of the samples to match most undetailed sample. Due to this, all the samples have the same accuracy of information. Moreover, the graphs describe the cell numbers as percentage from all the cells in sample. If there were no difference created between NFC and uncultured sample, the percentage of different cell types in random sample (if the sample size is big enough), should be approximately the same (obviously with some biological variation). However, some differences in the percentages can be seen, which means that the present matrices of interest have a true effect on the cell composition of the sample.

Seurat v3 was used for integration of the samples so that the batch effect between different samples would be minimized. The filtered gene-barcode-matrix was then normalized with Seurat v3. Logaritmic normalization opinion was used; feature counts of each cell were divided by total counts from that cell and multiplied by the scale factor. After this, each value was natural log-transformed with log1p.

Finally, the gene-barcode matrix was analyzed with PCA and Uniform Manifold Approximation and Projection was performed with top 20 principal components. Optimal number of PCA's were selected by using JackStraw-analysis and with visual representation of top genes in each cluster. The graph-based clustering with resolution 0.5 was performed following good practices of Seurat v3 library. Optimal resolution was obtained with clustree-package that allows us to observe stability and size of different clusters with altering resolution.

Individual clusters were then identified with conventional markers for each cell type. These markers and their mean expression and percentage of cells expressing cells in each cluster are provided in the supplementary.

### Materials

### INSTRUMENTS:

- Leica TCS SP8 MP CARS confocal laser scanning microscope with a HC PL APO 40x/1.10 water CS2 objective (Leica Microsystems)
- BD FACS Aria II Flow cytometer (BD Biosciences)
- Omega Fluostar microplate reader (BMG Labtech)

### SERVICES:

- Qubit, Biomedicum Functional Genomics Unit
- TapeStation, Biomedicum Functional Genomics Unit
- Bulk RNA sequencing, Biomedicum Functional Genomics Unit
- Chromium Single Cell Gene Expression analysis, FIMM Single-Cell Analytics unit
- BD Influx Cell sorter, Biomedicum Flow Cytometry Unit

### REAGENTS:

- NFC (LOT: 100 103 005)
- ANFC (LOT: 121 140 920)
- ANFC-A (LOT: 121 962 620)
- GrowDase (LOT: 180 020 820)
- AlexaFluor Phalloidin 546 (Invitrogen, A22283)
- ki67 (Abcam, ab15588)
- CC3 (Cell signaling, # 9661)
- CD11b (Abcam, ab8878)
- CD45 (Leica, NCL-L-LCA)
- IL-2 (2 ng/ml) (R&D Systems, BT202-025/CF)
- Matrigel Growth Factor Reduced Basement Membrane Matrix (Corning, 354230)
- Cultrex Organoid Harvesting Solution (Nordic Biosite, 3700-100-01)
- MammoCult growth media (STEMCELL, #05620)
- 8-well chamber slides (Thermo Fisher Scientific, 177402)
- Immu-Mount (Thermo Scientific, 9990402)
- Human DuoSet ELISA (R&D Systems; IL2: DY202-05, IL-10: DY217B-05, TNF-alpha: DY210-05, IFN-gamma: DY285B-05, IL-4: DY204-05, IL-6: DY206-05, IL-1 beta/IL1F2: DY201-05, LAP (TGF-beta 1): DY246-05)
- DuoSet Ancillary Reagent Kit 2 (R&D Systems, DY008)
- UltraComp eBeads Plus Compensation Beads (Invitrogen, 01-3333-42)
- Countbright Absolute counting beads (Invitrogen, C36950)
- SYTOX green (Thermo Fisher Scientific, S34860)
- Biotin (Sigma, B4639-1G)
- RNeasy Mini kit (Qiagen, 74106)
- Rna Clean & Concentrator^{™} kit (Zymo Research, BioSite-R1013)

**Table 3.**

| *Flow cytometry antibodies* | |
|---|---|
| **Antibody-conjugate** | **Clone & Manufacturer** |
| CD3-PerCP-Cy5.5 | Clone SK7; BD Biosciences 332771 |
| CD4-PE-Cy7 | Clone RPA-T4, BD Biosciences 560649 |
| CD8-BV510 | Clone SK1, BD Biosciences 563919 |
| CD19-PE-CF594 | Clone HIB19 (RUO), BD Biosciences 562321 |
| CD69-APC | Clone L78 (ASR), BD Biosciences 654663 |
| CD56-BV421 | Clone NCAM16.2, BD Biosciencs 562751 |
| CD3-PerCP-Cy5.5 | Clone SK7; BD Biosciences 332771 |
| CD56-BV421 | Clone NCAM16.2, BD Biosciencs 562751 |
| CD14-APC | Clone M5E2 (RUO), BD Biosciences 555399 |
| CD16-PE | Clone B73.1, BD Biosciences 561313 |
| CD80-BV510 | Clone L307.4, BD Biosciences 563084 |
| CD86-PE-Cy7 | Clone 2331, BD Biosciences 561128 |
| CD45-APC-H7 | Clone 2D1, BD Biosciences 560178 |

### Results

Wood-derived NFC matrices were found to be suitable for breast cancer explant *ex vivo* cultures. No increase in cell death was observed during 7 days of culture in any of the three matrices obtained from wood cellulose (chemically non-modified NFC (NFC), transparent oxidized anionic NFC (ANFC) or avidin-conjugated transparent oxidized anionic NFC (ANFC-A)) or in any of the tested concentrations (0.3% and 0.7/1.0%). NFC also supported immune cell viability and activation. The direct effects of NFC on immune cell populations were studied using a flow cytometry method with peripheral blood mononuclear cells (PBMCs; a population of T-cells, B-cells, NK-cells, and monocytes). Each of the studied matrices could support immune cell survival without the additional supplementation of IL-2, which is normally needed in their conventional growth at the suspension. All the investigated NFCs also supported the viability of patient-derived explant cultures (PDECs) in the 3D conditions and preserved the tumour resident immune cells. Cytokine secretion from both patient-derived tumours and PBMCs, however, revealed differences in the capacity of maintaining the immune cell types in the different matrices. At first, the ANFC-A matrix was observed to cause an inflammatory reaction both in PDECs as well as in PBMCs. This was evident from the production of inflammatory cytokines (IL-2, IFNγ, and IL-4) only in the ANFC-A compared to the other matrices. The reason for the inflammatory reaction was suspected to follow from the free avidin sites in the matrix, that would steal the biotin from the culture media and thus cause biotin deprivation to the cultures. To the test this hypothesis an additional biotin was supplemented in the culture media after the functionalization of the matrix. Adding biotin to the growth media clearly decreased inflammatory cytokine secretion into the media.

IL-2 was found to support immune cell survival and particularly the maintenance of tumour-infiltrating lymphocytes (TIL). Therefore, IL-2 was selected as a candidate to test matrix functionalization for immune oncological purposes. The functionalization of the ANFC-A with biotinylated IL-2 appeared to be a good choice for TIL maintenance, and a better survival of the immune cells was observed in the functionalized ANFC-A with the flow cytometric analysis. Particularly the NK and NKT cells appeared to respond most positively to IL-2 functionalization. The used IL-2 concentration was the same than used in the conventional floating cultures for PBMCs.

According to the flow cytometric analysis, of the nanocelluloses, T-cells were more prevalent in ANFC-A and ANFC in the higher polymer concentrations. Both matrices performed better in T-cell maintenance than NFC. With the monocytes a differential response to the polymer concentrations was shown. The CD14+ monocytes appeared to prefer the low concentrated matrices in general and IL-2 functionalization was not observed to give any growth benefit for these cells. On the contrary the CD14- monocytes responded positively to the IL-2 supplementation but did not show any preference on the matrix stiffness.

The results from the PDECs in general showed that the NFCs could retain the overall immune cell contexture of a patient sample and thus capture the patient specific immune cell profiles. Some differences in the capacity of maintaining the different immune cell type were observed between the different NFC matrices. One of these observations was the lower performance of the unmodified NFC compared to the ANFC-A and ANFC matrices.

To further confirm the results from the FACS, the activity of the tumour resident immune cells was investigated in PDECs in different NFCs, and the cytokine profiles from the 3D cultures were analyzed. Two major findings were observed. TNFα and IL-1β production was increased in the stiff matrices, whereas the IL-6 and TGFα production was increase in the soft matrices overtime. These observations were consistent with the results from the FACS analysis and confirmed the findings of the particular immune cell populations in different materials. When compared to the PBMCs in the same conditions the similar phenomena were not observed, which suggest an additional level of regulation on the immune cells coming from the tumour cells in the different NFC conditions.

To investigate further the dynamic between the tumour and tumour resident immune cells, a single cell RNA sequencing was performed for the original uncultured and the 3D cultured counterpart. The sequencing confirmed that the NFC cultured PDECs could retain the original immune cell composition of the tumour and retain the patient specific profiles. Additionally, slight changes in the immune cell contexture were observed. The biggest difference was observed in the myeloid lineage, which increased in the 0.3% NFC cultured explants compared to the original tumour. Simultaneously, the B-cell, mast cell, and T-cell populations were observed slightly to decrease.

Finally, the bulk RNA sequencing was performed to investigate the changes in the expressional profiles of the tumour cells, that could contribute the observed differences in the regulation of tumour resident immune cells compared to the PBMCs in the CMB cultures. The upregulation on multiple signalling pathways were observed in the NFCs and Matrigel in general compared to the original tumour. The upregulated gene signatures included e.g. following pathways: TGFβ, mTOR, KRAS, WNT. The only matrix, which failed to upregulate these pathways was 1.0% NFC, resembling thus most of the original uncultured sample by its expression profiles.

## Claims

1. An ex *vivo* tumour immune microenvironment model comprising patient-derived explant with a tumour-specific immune cell profile embedded in a matrix comprising nanofibrillar cellulose hydrogel having a concentration of the nanofibrillar cellulose in the range of 0.25-1.2% by weight, wherein the nanofibrillar cellulose comprises fibrils and/or fibril bundles having number-average diameter of 200 nm or less, such as 100 nm or less.

2. A method for preserving a tumour-specific immune cell profile in an ex *vivo* tumour immune microenvironment model, the method comprising
- providing a patient-derived explant with a tumour-specific immune cell profile,
- providing a matrix comprising nanofibrillar cellulose hydrogel, wherein the nanofibrillar cellulose comprises fibrils and/or fibril bundles having number-average diameter of 200 nm or less, such as 100 nm or less,
- embedding the patient-derived explant with a tumour-specific immune cell profile in the matrix comprising nanofibrillar cellulose hydrogel,
to obtain the ex *vivo* tumour immune microenvironment model, wherein the nanofibrillar cellulose hydrogel has a concentration of the nanofibrillar cellulose in the range of 0.25-1.2% by weight.

3. The method of claim 2, comprising incubating the patient-derived explant with a tumour-specific immune cell profile in a medium with one or more enzymes before embedding in the matrix comprising nanofibrillar cellulose hydrogel.

4. The method of claims 2 or 3, comprising culturing the patient-derived explant in the matrix comprising nanofibrillar cellulose hydrogel.

5. The ex *vivo* tumour tissue microenvironment model of claim 1 or the method of any of the claims 2-4, wherein the nanofibrillar cellulose comprises chemically anionically modified nanofibrillar cellulose.

6. The ex *vivo* tumour tissue microenvironment model of claim 1 or 5 or the method of any of the claims 2-5, wherein the nanofibrillar cellulose comprises TEMPO oxidized nanofibrillar cellulose.

7. The *ex vivo* tumour tissue microenvironment model of any of the claims 1 or 5-6 or the method of any of the claims 2-6, wherein the nanofibrillar cellulose, when dispersed in water, provides a zero shear viscosity in the range of 100-50000 Pa s, such as in the range of 5000-30000 Pa s, and a yield stress in the range of 1-50 Pa, such as in the range of 2-15 Pa, determined by rotational rheometer at a consistency of 0.5% by weight in aqueous medium at 22±1°C.

8. The *ex vivo* tumour tissue microenvironment model of any of the claims 1 or 5-7 or the method of any of the claims 2-7, wherein the nanofibrillar cellulose is chemically anionically modified nanofibrillar cellulose having a turbidity of 90 NTU or less, 60 NTU or less, or 40 NTU or less, for example from 3 to 90 NTU, such as from 5 to 60, for example 8-40, measured by nephelometry at a consistency of 0.1% (w/w) in aqueous medium.

9. The ex *vivo* tumour tissue microenvironment model of any of the claims 5-8 or the method of any of the claims 5-8, wherein the chemically anionically modified nanofibrillar cellulose is avidin-conjugated.

10. The ex *vivo* tumour tissue microenvironment model of claim 9 or the method of claim 9, wherein the nanofibrillar cellulose is functionalized with one or more functional molecules, such as with one or more cytokines.

11. The ex *vivo* tumour tissue microenvironment model of claim 10 or the method of claim 10, wherein the nanofibrillar cellulose is functionalized with interleukin IL-2.

12. The ex *vivo* tumour tissue microenvironment model of any of the claims 1-2 or 5-11 or the method of any of the claims 2-11, wherein the concentration of the nanofibrillar cellulose in the nanofibrillar cellulose hydrogel is in the range of 0.3-1.1% by weight.

13. Use of nanofibrillar cellulose comprising fibrils and/or fibril bundles having number-average diameter of 200 nm or less, such as 100 nm or less, for preparing the ex *vivo* tumour immune microenvironment model of any of the claims 1-2 or 5-12.

14. An *ex vivo* method for studying the effect of a substance of interest to tumour tissue obtained from a patient, such as cancer tissue, the method comprising
- providing the ex *vivo* tumour tissue microenvironment model of any of the claims 1-2 or 5-12,
- providing one or more substances of interest to the model,
- detecting the reaction of the model to the one or more substances of interest.

15. The ex *vivo* method for studying the effect of a substance of interest to tumour tissue obtained from a patient of claim 14, wherein the reaction of the model is a change in viability, a change in activation, maintenance of viability, and/or an indication of a change in viability and/or a change in activation of tumour cells and/or the immune cells.
